# EUROPEAN PATENT SPECIFICATION

(11) **EP 1 294 391 B1**
(45) Date of publication and mention of the grant of the patent: **15.08.2012**
(21) Application number: 01942118.9
(22) Date of filing: 08.06.2001
(51) Int. Cl.: A61K 38/17, A61K 39/395, C07K 16/28, A61P 37/06

(54) **COMBINATION OF AGENTS FOR INHIBITING TRANSPLANT REJECTION**
WIRKSTOFFKOMBINATION ZUR VERHINDERUNG DER TRANSPLANTATABSTOSSUNG
COMBINAISONS D'AGENTS POUR EVITER LE REJET DE GREFFE

(30) Priority: 09.06.2000 US 210671 P
(43) Date of publication of application: 26.03.2003
(73) Proprietor: Bristol-Myers Squibb Company, Princeton NJ 08543-4000 (US)
(72) Inventor: TOWNSEND, Robert, M., Boothwyn, PA 19061 (US); TODDERUD, Charles, Gordon, Newtown, PA 18940 (US); PEACH, Robert, J., San Diego, CA 92129 (US)
(74) Representative: Reitstötter - Kinzebach
(86) International application number: PCT/US2001/018619
(87) International publication number: WO 2001/095928

(56) References cited:
- WO-A-95/34320
- WO-A-98/56417
- H. SUN ET AL.: "Prevention of chronic rejection in mouse aortic allografts by combined treatment with CTLA4_ig and anti-CD40 ligand monoclonal antibody." TRANSPLANTATION, vol. 64, no. 12, 27 December 1997 (1997-12-27), pages 1838-1856, XP000971076 Baltimore, MD, USA
- D. SOUZA ET AL.: "Synergistic inhibition of established collagen induced arthritis (CIA) through dual inhibition of ICAM-1 and CD40L pathways." ARTHRITIS AND RHEUMATISM, vol. 42, September 1999 (1999-09), page S60 XP000987213 Philadelphia, PA, USA
- B. BLAZAR ET AL.: "Coblockade of the LFA1:ICAM and CD28/CTLA4:B7 pathways is a highly effective means of preventing acute lethal graft-versus-host disease induced by fully major histocompatibility complex-disparate donor grafts." BLOOD, vol. 85, no. 9, 1 May 1995 (1995-05-01), pages 2607-2618, XP000944558 New York, NY, USA

## Description

### FIELD OF THE INVENTION

The present invention relates to improved uses for regulating cell-mediated immune responses by disrupting at least three interactions between cell surface molecules and their natural ligands.

### BACKGROUND OF THE INVENTION

Acquired (specific) immunity is a stratagem used by a body to expand the repertoire of options available to combat antigenic challenge. Acquired immunity is mediated by lymphocytes, which are produced in the bone marrow by hematopoiesis. Activation of lymphocyte-mediated immunity in response to antigen recognition and binding result in activation of the two major subpopulations of lymphocytes: B lymphocytes (B-cells) and T lymphocytes (T-cells).

T- and B-cells are activated in an interdependent fashion. After antigenic challenge to a host, some host cells such as B-cells, macrophages and dendritic cells capture, internalize, and process the antigen for presentation on the cell surface. Then, after recognition and binding of the presented antigen by T-cells (specifically a subset of T-cells known as T-helper cells), the T-cells activate other T-cells as well as B-cells. In turn, activated B-cells stimulate resting T-cells. The complicated interactions between Band T-cells that regulate their activities are known to be mediated by several cell surface molecules.

Regulation of T-cell activation following recognition and binding of allo-antigen has been shown to require two distinct molecular signals mediated by cell surface molecules. The first signal is provided to the T-cell via antigen recognition and binding of the T-cell receptor, while the second signal is thought to be provided by recognition and binding of one or more of several putative receptor molecules on the surface of the T-cell by their ligands (1). To date, the most likely of these cell surface receptor molecules have been CD28 (2-5), CTLA4 (60-61) and CD154 (also known as CD40L or gp39) (6-9). The CD28 molecule is expressed on nearly all CD4+ T cells and approximately 50% of CD8+ T cells (4). The ligands for CD28 have been shown to be the B7-1 and B7-2 (also known as CD80 and CD86, respectively) molecules expressed on the surface of antigen presenting cells such as B cells (4; 5; 10). Interaction between CD28 and the B7 molecules stimulates the activation of T-cells.

The B7 molecules are also ligands for the cell surface receptors, CTLA4, which are present on activated T-cells. Interaction between CTLA4 and B7 induces a state of anergy in T-cells, counteracting CD28/B7 induced activation of T-cells.

The interaction of CD28 and/or CTLA4 with B7 ligands can be efficiently blocked by soluble CTLA4Ig (DNA encoding CTLA4Ig was deposited on May 31, 1991 with American Type Culture Collection (ATCC), .10801 University Blvd., Manasas, VA 20110-2209, with ATCC identification number 68629; and CTLA4Ig-24, a Chinese Hamster Ovary (CHO) cell line expressing CTLA4Ig was deposited on May 31, 1991 with ATCC identification number CRL-10762), a fusion protein with a higher avidity for B7-1 and B7-2 than CD28 (11-13). Numerous studies have demonstrated that blockade of this receptor pathway leads to inhibition of antigen activation of T-cells both *in vitro* (13; 14) and *in vivo* (12; 15; 16).

The CD154 molecule is predominantly expressed on activated T-cells while CD40 is expressed on antigen presenting cells such as B cells. The interaction between CD154 and CD40 activates B-cells and more T-cells. Blockade of the CD40/CD154 pathway by monoclonal antibodies has also proven to be an effective method of inhibiting lymphocyte-mediated immune responses *in vitro* (17; 18) and *in vivo* (19-21.).

Although CD28, CTLA4 and CD 154 have been the most studied lymphocyte receptors, other cell surface molecules have been implicated in the cell regulation process. Some of these molecules include 4-1BB, ICOS, CD99 and several adhesion molecules from the integrin family, such as Lymphocyte function associated antigen-1 (LFA-1) (22-33).

The LFA-1 molecule is formed by the combination of the integrin αL subunit (CD11a) with the integrin β2 subunit (CD18). LFA-1 is expressed on numerous leukocyte cell types including lymphocytes (e.g. T-cells), granulocytes, monocytes, macrophages, etc., and has been described in detail as mediating cell-cell and cell-matrix interactions via its interaction with various ligands, including molecules from the ICAM family (e.g. ICAM-1, ICAM-2 and ICAM-3). Other ligands of the LFA-1 β2 subunit (CD18) include α-actinin, filamin, and cytohesin-1. The function of LFA-1 as an adhesion molecule, augmenting the interaction between LFA-1-positive cells and LFA-1 binding ligands such as ICAM-1 on leukocytes, epithelial cells and endothelium, has been long recognized; however, more recently, evidence has emerged implicating LFA-1 in the signaling process of T cells following antigen recognition (26; 27; 34-38).

Following allogeneic organ or tissue transplantation, cell-mediated graft rejection is still a major obstacle to successful long-term graft survival. The use of monoclonal antibodies that block the LFA-1/ICAM-1 interaction has been shown to prolong graft survival (39-44). In addition, agents that block lymphocytic interactions such as CTLA4Ig and anti-CD40/CD154 monoclonal antibodies, have recently been demonstrated to be efficacious against the graft rejection process in multiple animal models including non-human primates (14; 45-51). Furthermore, combining both CD28 and CD40/CD154 blockade dramatically improved graft survival leading to long-term graft acceptance and allogeneic hypo-responsiveness in some animal models of transplantation (52-54), and is described to be useful for treating autoimmune diseases and allergy (91). Yet, other models of allogeneic transplantation, such as BALB/c->C57BL/6 murine skin transplants, have been shown to be resistant to CD28+CD 154 blockade (55), suggesting that other signaling pathways may be involved in allograft recognition.

The use of a combination of two agents - one inhibiting the interaction of CD28 and/or CTLA4 with B7, the other inhibiting the T-cell adhesion to antigen-presenting cells - for inhibiting an antigen-specific T-cell mediated immune response is described in reference 92.

Dual inhibition of ICAM-1 and CD40L pathways has been reported to inhibit collagen induced arthritis in a mouse model (90). Blocking both the CD28/CTLA4:B7 and the LFA1:ICAM pathways was reported as a means for preventing acute lethal graft-versus-host disease in mice (58).

Presently, there exists a need to provide ways to regulate cell-mediated immune responses after antigen presentation, for example to suppress graft (e.g., allograft) rejection after transplantation of tissues, so as to increase the survival rate of the transplanted tissue.

The inhibition of immune responses resulting from the blockade of CD28/B7, CTLA4/B7 and/or CD40/CD154 signals is potent, but incomplete in some cases. The results presented herein demonstrate that blockade of these pathways, in addition to blockade of the LFA-1 pathway, unexpectedly enhances graft survival and regulates the cell-mediated immune response to antigen presentation.

### SUMMARY OF THE INVENTION

The invention disclosed herein provides combinations of agents blocking the interaction of cell surface molecules such as CD28, CTLA4, B7, CD40, CD154 and adhesion molecules such as LFA-1, with their natural ligands for use in inhibiting transplant rejection as set forth in claims 1 to 4. The invention herein involves the discovery that blockade of these molecules provides improved methods to promote long-term survival of transplants. Addition of an agent directed against LFA-1 greatly enhances allogeneic graft survival in mice treated with an agent directed against B7 (e.g. soluble CTLA4 molecules) and an agent against CD 154 (e.g. anti-CD 154 monoclonal antibody) for both murine skin and cardiac transplants. Use of a combination of three agents, for example, directed against each of CD28/CTLA4/B7, CD40/CD154 and LFA-1/ICAM pathways, respectively, also enhances murine skin graft survival.

### BRIEF DESCRIPTION OF THE FIGURES

Figure 1 is a graph showing the effect of soluble CTLA4-Ig and monoclonal antibodies MR1 and anti-LFA-1 on skin graft rejection rates, as described in Example 1, *infra.*
Figure 2 is a graph showing the effect of soluble CTLA4-Ig and monoclonal antibodies MR1 and anti-LFA-1 on heart graft rejection rates, as described in Example 2, *infra.*
Figure 3 show the percentage of myocardium remaining after transplantation and therapy with soluble CTLA4-Ig and monoclonal antibodies MR1 and anti-LFA-1, in a murine heterotropic heart transplant model, as described in Example 2, *infra.*
Figure 4 shows inflammation severity scores after transplantation and therapy with soluble CTLA4-Ig and monoclonal antibodies MR1 and anti-LFA-1, in a murine heterotropic heart transplant model, as described in Example 2, *infra.*
Figure 5 shows the amino acid and nucleic acid sequence of human CTLA4Ig with a leader sequence attached to the N-terminus of the molecule, as described in Example 3, *infra*.
Figure 6 shows the amino acid and nucleic acid sequence of human L104EA29YIg with a leader sequence attached to the N-terminus of the molecule, as described in Example 3, *infra*
Figure 7 are an SDS gel (FIG. 7A) for CTLA4Ig (lane 1), L104EIg (lane 2), and L104EA29YIg (lane 3A); and size exclusion chromatographs of CTLA4Ig (FIG. 7B) and L104EA29YIg (FIG. 7C).
Figures 8A and 8B illustrate a ribbon diagram of the CTLA4 extracellular Ig V-like fold generated from the solution structure determined by NMR spectroscopy. FIG. 8B shows an expanded view of the S25-R33 region and the MYPPPY region indicating the location and side-chain orientation of the avidity enhancing mutations, L104 and A29.
Figures 9A & 9B illustrate data from FACS assays showing binding of L104EA29YIg, L104EIg, and CTLA4Ig to human CD80- or CD86-transfected CHO cells as described in Example 3, infra.
Figures 10A & 10B depicts inhibition of proliferation of CD80-positive and CD86-positive CHO cells as described in Example 3, infra.
Figures 11A & 11B shows that L104EA29YIg is more effective than CTLA4Ig at inhibiting proliferation of primary and secondary allostimulated T cells as described in Example 3, infra.
Figures 12A-C illustrate that L104EA29YIg is more effective than CTLA4Ig at inhibiting IL-2 (FIG. 12A), IL-4 (FIG. 12B), and γ-interferon (FIG. 12C) cytokine production of allostimulated human T cells as described in Example 3, infra.
Figure 13 demonstrates that L104EA29YIg is more effective than CTLA4Ig at inhibiting proliferation of phytohemaglutinin- (PHA) stimulated monkey T cells as described in Example 3, infra.

### DETAILED DESCRIPTION OF THE INVENTION

### Definitions

As used in this application, the following words or phrases have the meanings specified.

As used herein, "ligand" refers to a molecule that recognizes and binds another molecule.

As used herein, "regulate" means to inhibit or stimulate a response, for example "regulating a lymphocyte-mediated immune response" means to inhibit or stimulate a lymphocyte associated immune response.

As used herein, "receptor" means a molecule that, when bound by a ligand, instigates an intracellular pathway cascade leading to an altered cell state. Receptors may be found on several cell types including lymphocytes.

As used herein, to "block" or "inhibit" a receptor, signal or molecule means to interfere with the activation of the receptor, signal or molecule, as detected by an art-recognized test. For example, blockage of a cell-mediated immune response can be detected by determining enhancement of allogeneic graft survival. Blockage or inhibition may be partial or total.

As used herein, "inhibit cell-cell" or "cell-matrix adhesion" means to prevent the interaction between a cell surface adhesion molecule and its ligand on another cell or in the extracellular matrix. Examples of adhesion molecules and ligands include, but are not limited to, LFA-1 (also known as CD11a/CD18 or as integrin αLβ2), Mac-1 (CD11b/CD18), p150,95 (CD11c/CD18), ICAM-1 (CD54), ICAM-2, ICAM-3, VLA-1, CD44, CD62 (E, L and P), CD106, fibrinogen, α-actinin, filamin and cytohesin-1.

As used herein, a "portion" or "fragment" of a molecule means any part of the intact molecule that retains binding activity. For example, a "fragment of CTLA4" or "portion of CTLA4" is the extracellular domain of CTLA4 or segment thereof that recognizes and binds its target, e.g. B7.

As used herein a "derivative" is a molecule that shares sequence homology and activity of its parent molecule. For example, a derivative of CTLA4 includes a soluble CTLA4 molecule, or a soluble CTLA4 mutant molecule, having an amino acid sequence at least 70% similar to the extracellular domain of wildtype CTLA4, and which recognizes and binds B7. An example of a soluble CTLA4 molecule is CTLA4Ig (deposited on May 31, 1991 with the American Type Culture Collection (ATCC) 10801 University Blvd., Manasas, VA 20110-2209. ATCC identification number 68629). CTLA4Ig-24, a Chinese Hamster Ovary (CHO) cell line expressing CTLA41g was deposited on May 31, 1991 with ATCC identification number CRL-10762. An example of a soluble CTLA4 mutant molecule is L104EA29YIg (deposited on June 19, 2000 with ATCC identification number PTA-2104).

As used herein, "agent" refers to a molecule that is used to regulate a cell-mediated immune response by blocking molecules mediating the immune response.

As used herein, "first agent" is a molecule, or a portion or portions thereof, that recognizes and binds the CD28, CTLA4 or B7 molecules on lymphocytes. According to the invention, the "first agent" is a soluble CTLA4 molecule (e.g. soluble CTLA4 mutant molecule).

As used herein, "second agent" is a molecule, or a portion or portions thereof, that recognizes and binds CD40 or CD154 (also known as CD40L or as gp39) on lymphocytes. According to the invention, "second agent" is an anti-CD154 monoclonal antibody.

As used herein, "third agent" is a molecule, or a portion or portions thereof, that interferes with LFA-1 adhesion to its ligands such as ICAM-1, ICAM-2, ICAM-3, α-actinin, filamin or cytohesion-1. According to the invention, "third agent" is an anti-LFA-1 monoclonal antibody

As used herein, "B7" refers to B7 family members including B7-1 (also known as CD80), B7-2 (also known as CD86) and B7-3 molecules that may recognize and bind CD28 and/or CTLA4.

As used herein "wild type CTLA4" has the amino acid sequence of naturally occurring, full length CTLA4 (U.S. Patent Nos. 5,434,131, 5,844,095, 5,851,795), or the extracellular domain threreof, which binds a B7, and/or interferes with a B7 from binding their ligands. In particular embodiments, the extracellular domain of wild type CTLA4 begins with methionine at position +1 and ends at aspartic acid at position +124, or the extracellular domain of wild type CTLA4 begins with alanine at position -1 and ends at aspartic acid at position +124. Wild type CTLA4 is a cell surface protein, having an N-terminal extracellular domain, a transmembrane domain, and a C-terminal cytoplasmic domain. The extracellular domain binds to target antigens, such as a B7. In a cell, the naturally occurring, wild type CTLA4 protein is translated as an immature polypeptide, which includes a signal peptide at the N-terminal end. The immature polypeptide undergoes post-translational processing, which includes cleavage and removal of the signal peptide to generate a CTLA4 cleavage product having a newly generated N-terminal end that differs from the N-terminal end in the immature form. One skilled in the art will appreciate that additional post-translational processing may occur, which removes one or more of the amino acids from the newly generated N-terminal end of the CTLA4 cleavage product. The mature form of the CTLA4 molecule includes the extracellular domain of CTLA4, or any portion thereof, which binds to B7.

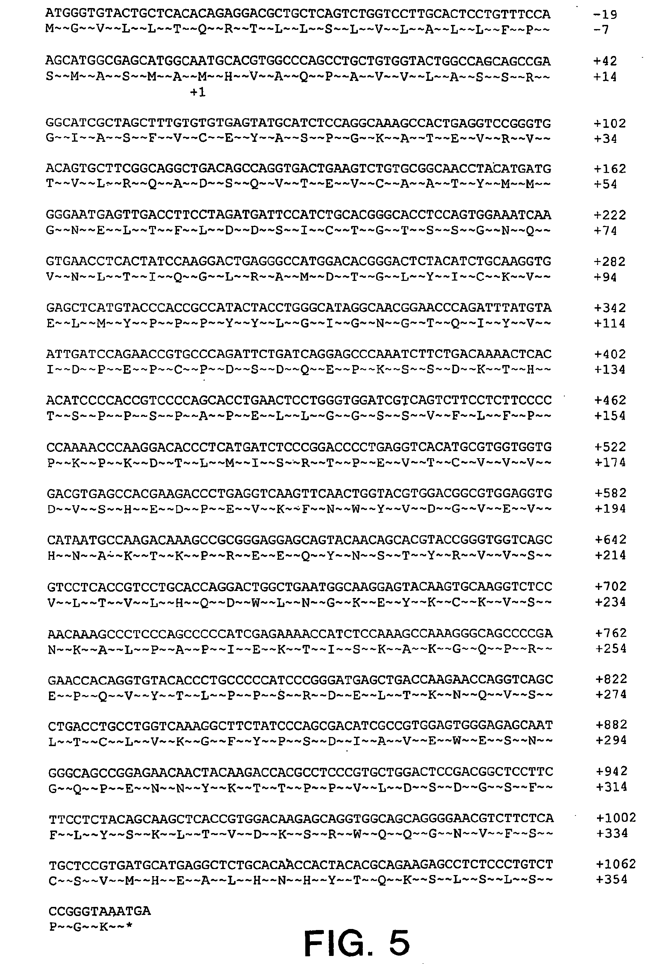

"CTLA4Ig" is a soluble fusion protein comprising an extracellular domain of wild type CTLA4, or a portion thereof that binds a B7, joined to an Ig tail. A particular embodiment comprises the extracellular domain of wild type CTLA4 starting at methionine at position +1 and ending at aspartic acid at position +124; or starting at alanine at position -1 to aspartic acid at position +124; a junction amino acid residue glutamine at position +125; and an immunoglobulin portion encompassing glutamic acid at position +126 through lysine at position +357 (Figure 5).

As used herein, a "fusion protein" is defined as one or more amino acid sequences joined together using methods well known in the art and as described in U.S. Pat. No. 5,434,131 or 5,637,481. The joined amino acid sequences thereby form one fusion protein.

As used herein, "soluble" refers to any molecule, or fragments and derivatives thereof, not bound or attached to a cell i.e. circulating. For example, CTLA4, B7 or CD28 can be made soluble by attaching an immunoglobulin (Ig) moiety to the extracellular domain of CTLA4, B7 or CD28, respectively. Alternatively, a molecule such as CTLA4 can be rendered soluble by removing its transmembrane domain. The soluble molecules used in the methods of the invention may or may not include a signal (or leader) sequence. Typically, the molecules do not include a leader sequence.

As used herein, "soluble CTLA4 molecules" means circulating or non-cell-surface-bound CTLA4 molecules or any functional portion of a CTLA4 molecule that binds B7 including, but not limited to: CTLA4Ig fusion proteins, wherein the extracellular domain of CTLA4 is fused to an immunoglobulin (Ig) moiety rendering the fusion molecule soluble, or fragments and derivatives thereof; proteins with the extracellular domain of CTLA4 fused or joined with a portion of a biologically active or chemically active protein such as the papillomavirus E7 gene product (CTLA4-E7), melanoma-associated antigen p97 (CTLA4-p97) or HIV env protein (CTLA4-env gp120), or fragments and derivatives thereof; hybrid (chimeric) fusion proteins such as CD28/CTLA4Ig, or fragments and derivatives thereof; CTLA4 molecules with the transmembrane domain removed to render the protein soluble, or fragments and derivatives thereof. "Soluble CTLA4 molecules" also include fragments, portions or derivatives thereof, and soluble CTLA4 mutant molecules, having CTLA4 binding activity. The soluble CTLA4 molecules used in the methods of the invention may or may not include a signal (or leader) sequence. Typically, the molecules do not include a leader sequence.

As used herein, a "CTLA4 mutant molecule" means wildtype CTLA4 or portions thereof (derivatives or fragments) that have a mutation or multiple mutations (preferably in the extracellular domain of wildtype CTLA4). A CTLA4 mutant molecule has a sequence that it is similar but not identical to the sequence of wild type CTLA4 molecule, but still binds a B7. The mutations may include one or more amino acid residues substituted with an amino acid having conservative (e.g., substitute a leucine with an isoleucine) or nonconservative (e.g., substitute a glycine with a tryptophan) structure or chemical properties, amino acid deletions, additions, frameshifts, or truncations. Mutant CTLA4 molecules may include a non-CTLA4 molecule therein or attached thereto e.g., the extracellular domain of CTLA4, or portions or fragments thereof, joined to an immunoglobulin constant domain, resulting in the CTLA4Ig molecule (ATCC 68629) or the L104EA29YIg molecule (ATCC PTA-2104) which are copending in U. S. Patent Application Serial Numbers 60/287,576 and 60/214,065. The mutant molecules may be soluble (i.e., circulating), or bound to a cell surface. Additional CTLA4 mutant molecules include those described in U. S. Patent Application Serial Numbers 09/865,321, 60/214,065 and 60/287,576; and in U. S. Patent Numbers 6,090,914, 5,844,095 and 5,773,253. CTLA4 mutant molecules can be made synthetically or recombinantly.

As those skilled-in-the-art will appreciate, mutations in a nucleotide sequence may or may not result in a change in the amino acid sequence. In that regard, certain codons encode the same amino acid. Examples include codons CGT, CGG, CGC, and CGA encoding the amino acid, arginine (R); or codons GAT, and GAC encoding the amino acid, aspartic acid (D). Thus, a protein can be encoded by one or more nucleic acid molecules that differ in their specific nucleotide sequence, but still encode protein molecules having identical sequences. The amino acid coding sequence is as follows:

| **Amino Acid** | **Symbol** | **One Letter** **Symbol** | **Codons** |
|---|---|---|---|
| Alanine | Ala | A | GCU, GCC, GCA, GCG |
| Cysteine | Cys | C | UGU, UGC |
| Aspartic Acid | Asp | D | GAU, GAC |
| Glutamic Acid | Glu | E | GAA, GAG |
| Phenylalanine | Phe | F | UUU, UUC |
| Glycine | Gly | G | GGU, GGC, GGA, GGG |
| Histidine | His | H | CAU, CAC |
| Isoleucine | Ile | I | AUU, AUC, AUA |
| Lysine | Lys | K | AAA, AAG |
| Leucine | Leu | L | UUA, UUG, CUU, CUC, CUA, CUG |
| Methionine | Met | M | AUG |
| Asparagine | Asn | N | AAU, AAC |
| Proline | Pro | P | CCU, CCC, CCA, CCG |
| Glutamine | Gln | Q | CAA, CAG |
| Arginine | Arg | R | CGU, CGC, CGA, CGG, AGA, AGG |
| Serine | Ser | S | UCU, UCC, UCA, UCG, AGU, AGC |
| Threonine | Thr | T | ACU, ACC, ACA, ACG |
| Valine | Val | V | GUU, GUC, GUA, GUG |
| Tryptophan | Trp | W | UGG |
| Tyrosine | Tyr | Y | UAU, UAC |

As used herein "the extracellular domain of CTLA4" is any portion of CTLA4 that recognizes and binds a B7. For example, an extracellular domain of CTLA4 comprises methionine at position +1 to aspartic acid at position +124 (Figure 5). Alternatively, an extracellular domain of CTLA4 comprises alanine at position -1 to aspartic acid at position +124 (Figure 5). The extracellular domain includes fragments or derivatives of CTLA4 that bind a B7.

As used herein, "lymphocyte" refers to mononuclear cells that mediate humoral- or cell-mediated immunity. Major subsets of lymphocytes include B and T cells.

As used herein, "immune system diseases" refer to autoimmune, immunoproliferative disorders and graft-related disorders including, but not limited to: graft-versus-host disease (GVHD) (e.g., such as may result from bone marrow transplantation, or in the induction of tolerance); immune disorders associated with graft transplantation rejection (e.g. chronic rejection, tissue or cell allo- or xenografts including solid organs, skin, islets, muscles, hepatocytes, neurons, etc.); T cell lymphoma; psoriasis; T cell acute lymphoblastic leukemia; testicular angiocentric T cell lymphoma; benign lymphocytic angiitis; and autoimmune diseases such as lupus (e.g. lupus erythematosus, lupus nephritis), Hashimoto's thyroiditis, primary myxedema, Graves' disease, pernicious anemia, autoimmune atrophic gastritis, Addison's disease, diabetes (e.g. insulin dependent diabetes mellitis, non-insulin dependent diabetes), good pasture's syndrome, myasthenia gravis, pemphigus, Crohn's disease, sympathetic ophthalmia, autoimmune uveitis, multiple sclerosis, autoimmune hemolytic anemia, idiopathic thrombocytopenia, primary biliary cirrhosis, chronic action hepatitis, ulceratis colitis, Sjogren's syndrome, rheumatic diseases (e.g. rheumatoid arthritis), polymyositis, scleroderma, and mixed connective tissue disease.

As used herein, "subject" means any living organism to which the agents can be administered in order to regulate an immune response. Subjects may include, but are not limited to, humans, monkeys, mice, rats, cats, dogs, hamsters, any transgenic animals, any allograft recipients, any xenograft recipients or any graft recipients.

As used herein, "administer" means to provide an agent to a subject by any convenient method, including, but not limited to, oral administration, inhalation administration, intravenous administration, intraperitoneal administration, subcutaneous administration, intramuscular administration, administration by suppositories or topical contact, or administration by slow release devices such as vesicles or capsules.

As used herein, "gene therapy" is a process to treat a disease by genetic manipulation so that a sequence of nucleic acid is transferred into a cell, the cell then expressing any genetic product encoded by the nucleic acid. For example, as is well known by those skilled in the art, nucleic acid transfer may be performed by inserting an expression vector containing the nucleic acid of interest into cells *ex vivo* or *in vitro* by a variety of methods including, for example, calcium phosphate precipitation, diethyaminoethyl dextran, polyethylene glycol (PEG), electroporation, direct injection, lipofection or viral infection (63, 64, 65). Alternatively, nucleic acid sequences of interest may be transferred into a cell *in vivo* in a variety of vectors and by a variety of methods including, for example, direct administration of the nucleic acid into a subject (66), or insertion of the nucleic acid into a viral vector and infection of the subject with the virus (67-70). Other methods used for *in vivo* transfer include encapsulation of the nucleic acid into liposomes, and direct transfer of the liposomes, or liposomes combined with a hemagglutinating Sendai virus, to a subject (71). The transfected or infected cells express the protein products encoded by the nucleic acid in order to ameliorate a disease or the symptoms of a disease. The expressed protein products may be secreted or remain in the transfected or infected cells.

As used herein, "pharmaceutically acceptable carrier" means any material that may be combined with the agents in order to administer the agents to a subject in any form. For example, a carrier includes any material that will maintain the agents' effective activity when administered to a subject and that is non-reactive with a subject's immune system.

Potential carriers may include, but are not limited to, any solvents, media, suspensions, emulsions or other excipients such as starch, milk, sugar, certain types of clay, gelatin, stearic acids, stearate salts, talcum, oils, gums, glycols, flavorings, perservatives or color additives, etc. Potential carrier forms may include sterile solutions, aerosols, liposomes, vesicles, suppositories, pills, tablets or capsules.

In order that the invention herein described may be more fully understood, the following description is set forth.

### USES OF THE INVENTION

Cell-mediated immune responses can be regulated by blocking interactions between receptors on lymphocytes such as CTLA4, CD28, B7, CD40 or CD 154, with their ligands and also by blocking LFA-1-mediated interactions. This involves: blocking a first lymphocytic signal by contacting a molecule such as CD28, CTLA4 or B7 on a lymphocyte with a first agent; blocking a second lymphocytic signal by contacting a molecule such as CD40 or CD154 on a lymphocyte with a second agent; and blocking adhesion molecule mediated interactions with its ligands by contacting an adhesion molecule (e.g. LFA-1) or the ligands of the adhesion molecule with a third agent.

Further, an immune system disease can be treated by blocking interactions between receptors on lymphocytes such as CTLA4, CD28, B7, CD40 or CD154, with their ligands and also by blocking LFA-1-mediated interactions. For this purpose, at least three agents are administered: a first agent which blocks a CD28/CTLA4/B7-mediated signal by binding CD28, CTLA4 or B7; a second agent which blocks a CD40/CD154-mediated signal by binding either CD40 or CD154; and a third agent which blocks an adhesion molecule-mediated interaction by binding to LFA1, ICAM-1, ICAM-2, ICAM-3, α-actinin, filamin or cytohesin-1. Blockage by a combination of any of the three agents therapeutically treating an immune system related disease.

The first agent acts by interfering with the interaction between a receptor on a lymphocyte (e.g., CD28 and/or CTLA4) and its ligand (e.g., B7-1 and/or B7-2). The first agent is a soluble form (or portion or derivative thereof) of the receptor, namely soluble CTLA4. In a preferred embodiment, the first agent is a soluble CTLA4 molecule, such as CTLA4Ig (ATCC68629) or L104EA29YIg (ATCC PTA2104).

The second agent acts by interfering with the interaction between a second receptor on a lymphocyte (e.g., CD154) and its ligand (e.g., CD40). The second agent is an antibody that recognizes and binds the second receptor, namely an anti-CD154 monoclonal antibody. In a preferred embodiment, the second agent is an anti-CD154 (e.g. MR1 as described in reference 56, ATCC HB-10916, ATCC HB-12055 and ATCC HB-12056).

The third agent interferes with adhesion molecule LFA-1 (CD11a/CD18) interactions with its ligands. Said ligands include, but are not limited to, LFA-1, α-actinin, filamin and cytohesin-1. LFA-1 ligands such as Imam-1, ICAM-2, ICAM-3, α-actinin, filamin and cytohesin-1, etc., can be located on another cell or in the extracellular matrix. The third agent is an antibody that recognizes and binds the adhesion molecule LFA-1. In a preferred embodiment, the third agent is an anti-LFA-1 monoclonal antibody such as ATCC HB-9579, and ATCC TIB-213.

The invention allows to regulate a cell-mediated immune response by blocking at least three cellular pathways: the CD28/CTLA4B7-mediated pathway with a first agent, CTLA4Ig; the CD40/CD154 mediated pathway with a second agent, anti-CD 154 monoclonal antibody MR1; and an adhesion molecule mediated pathway with a third agent, anti-LFA-1 monoclonal antibody M17/5.2.

Further described herein are pharmaceutical compositions for use in the treatment of immune system diseases comprising pharmaceutically effective amounts of the three agents described above. Accordingly, a cell-mediated immune response to antigen challenge can be regulated by application of a combination of at least three agents to a subject in order to disrupt at least three cellular contacts. Subjects may include, but are not limited to, humans, monkeys, mice, rats, cats, dogs, hamsters, etc. The compositions may additionally include other therapeutic agents, including, but not limited to, drug toxins, enzymes, antibodies (or portions or derivative thereof), or conjugates.

The administration of the agents may occur at the same time or at different times. For example, the agents can be administered in a specified order and can be applied concurrently or sequentially in a time-dependant application or any combination thereof. The agents may be administered sequentially or concurrently and in any order. A subject may be treated with the agents before, during, or after (or any combination thereof) an immune response to antigen presentation depending on the dosage and mode of application.

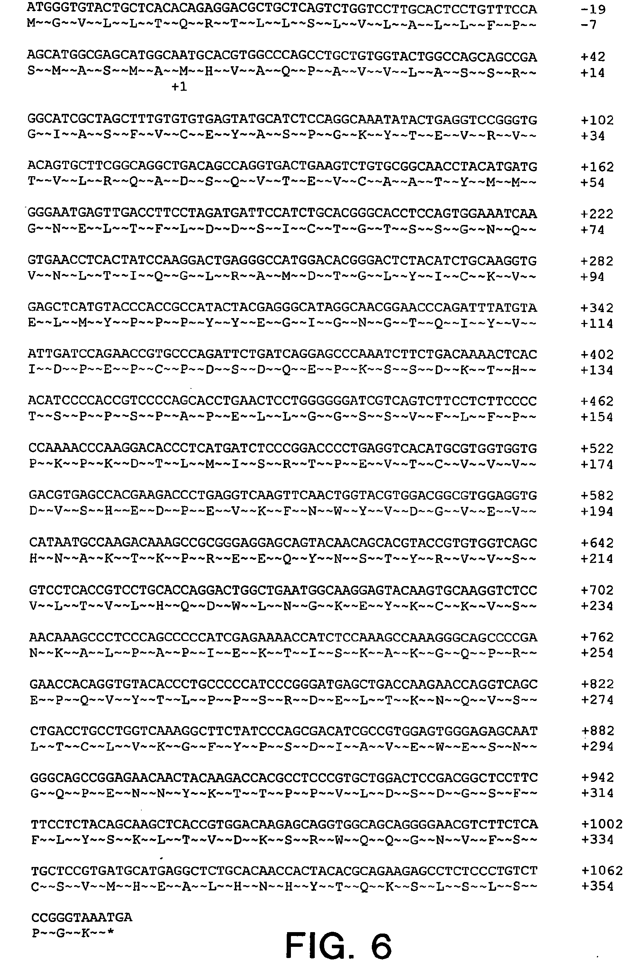

Dosage of the agents is dependant upon many factors including, but not limited to, the type of subject (i.e. the species), the agent used (e.g. CTLA4Ig or L104EA29YIg), location of the antigenic challenge, the type of tissue affected, the type of immune system disease being treated, the severity of the disease, a subject's health and response to the treatment with the agents. Accordingly, dosages of the agents can vary depending on each subject, agent and the mode of administration. For example, soluble CTLA4 molecules such as L104EA29YIg (included in Figure 6; as encoded by DNA deposited with ATCC accession number PTA-2104; and as described in U. S. Patent Application Serial Numbers 09/579,927, 60/287,576 and 60/214,065) may be administered in an amount between 0.1 to 20.0 mg/kg weight of a human subject/day, preferably between 0.5 to 10.0 mg/kg/day.

Administration of the agents may be performed in many permissible ways including, but not limited to: injection (e.g. intravenous, intraperitoneal, intramuscular, etc.), oral administration, inhalation, topical contact, gene therapy, administration by a mechanical release device such as a pump, administration of slow release devices such as vesicles or capsules, or suppositories. Depending on the means of administration, the agents may be compounded with pharmaceutically acceptable carriers for convenient application and effective use of the agents.

The pharmaceutical compositions also preferably include suitable carriers and adjuvants which include any material which when combined with the molecule of the invention (e.g., a soluble CTLA4 mutant molecule, such as, L104EA29Y or L104E) retains the molecule's activity and is non-reactive with the subject's immune system. Examples of suitable carriers and adjuvants include, but are not limited to, human serum albumin; ion exchangers; alumina; lecithin; buffer substances, such as phosphates; glycine; sorbic acid; potassium sorbate; and salts or electrolytes, such as protamine sulfate. Other examples include any of the standard pharmaceutical carriers such as a phosphate buffered saline solution; water; emulsions, such as oil/water emulsion; and various types of wetting agents. Other carriers may also include sterile solutions; tablets, including coated tablets and capsules. Typically such carriers contain excipients such as starch, milk, sugar, certain types of clay, gelatin, stearic acid or salts thereof, magnesium or calcium stearate, talc, vegetable fats or oils, gums, glycols, or other known excipients. Such carriers may also include flavor and color additives or other ingredients. Compositions comprising such carriers are formulated by well known conventional methods. Such compositions may also be formulated within various lipid compositions, such as, for example, liposomes as well as in various polymeric compositions, such as polymer microspheres.

Administration of the agents in any form to regulate a cell-mediated immune response in turn can affect the development of immune system diseases such as immunoproliferative diseases or autoimmune diseases/disorders of an immune system. Examples of immune system diseases include, but are not limited to, graft versus host disease (GVHD), psoriasis, immune disorders associated with graft transplant rejection (e.g. allograft transplant rejection), T cell lymphoma, T cell acute lymphoblastic leukemia, testicular angiocentric T cell lymphoma, benign lymphocytic angiitis, lupus (e.g. lupus erythematosus, lupus nephritis), Hashimoto's thyroiditis, primary myxedema, Graves' disease, pernicious anemia, autoimmune atrophic gastritis, Addison's disease, diabetes (e.g. insulin dependent diabetes mellitis, type I diabetes mellitis), good pasture's syndrome, myasthenia gravis, pemphigus, Crohn's disease, sympathetic ophthalmia, autoimmune uveitis, multiple sclerosis, autoimmune hemolytic anemia, idiopathic thrombocytopenia, primary biliary cirrhosis, chronic action hepatitis, ulceratis colitis, Sjogren's syndrome, rheumatic diseases (e.g. rheumatoid arthritis), polymyositis, scieroderma, and mixed connective tissue disease.

Further described herein are methods for treating immune system diseases in a subject. Examples of immune system diseases are as described *supra* and include autoimmune disorders. Many autoimmune disorders result from inappropriate activation of T lymphocytes that are reactive against autoantigens, and which promote the production of cytokines and autoantibodies that are involved in the pathology of the disease. Administration of the three agents of the invention to a subject suffering from or susceptible to an autoimmune disorder may prevent the activation of autoreactive T cells and may reduce or eliminate disease symptoms.

In a preferred embodiment of the invention, administration of a combination of at least three agents, CTLA4Ig, anti-CD154 monoclonal antibody and anti-LFA-1 monoclonal antibody, regulates an immune response as monitored by allograft transplant survival assays.

The present invention further provides the combination of agents described herein for use in inhibiting allograft transplant rejections by a subject. Typically, in transplants, rejection of the graft is initiated through its recognition as foreign by T cells, followed by an immune response that destroys the graft. The three agents of the combination of agents described herein, by inhibiting the interaction of cell surface molecules with their ligands, block immune responses subsequent to allograft transplantation. Examples of immune responses affected by administration of the agents include inhibition of T lymphocyte proliferation and/or cytokine secretion that may result in reduced tissue destruction and induction of antigen-specific T cell unresponsiveness in long-term graft acceptance without the need for generalized immunosuppression. The invention thus enhances allograft survival.

Furthermore, the agents of the combination of agents described herein can be administered with other pharmaceuticals including, but not limited to, other drugs. For example, it may be used in combination with a calcineurin inhibitor, e.g. cyclosporin A or FK506; an immunosuppressive macrolide, e.g. rapamycine or a derivative thereof; e.g. 40-O-(2-hydroxy)ethylrapamycin, a lymphocyte homing agent, e.g. FTY720 or an analog thereof; corticosteroids; cyclophosphamide; azathioprene; methotrexate; leflunomide or an analog thereof; mizoribine; mycophenolic acid; mycophenolate mofetil; 15-deoxyspergualine or an analog thereof; immunosuppressive monoclonal antibodies (or portions or derivative thereof), e.g., monoclonal antibodies to leukocyte receptors (or portions or derivative thereof), e.g., MHC, CD2, CD3, CD4, CD IIa/CD18, CD7, CD25, CD 27, B7, CD40, CD45, CD58, CD 137, ICOS, CD 150 (SLAM), OX40, 4-1BB or their ligands; or other immunomodulatory compounds, e.g. Selectin antagonists and VLA-4 antagonists.

Where the soluble CTLA4 mutant molecules described herein are administered in conjunction with other immunosuppressive/immunomodulatory or anti-inflammatory therapy, e.g. as hereinabove specified, dosages of the co-administered immunosuppressant, immunomodulatory or anti-inflammatory compound will of course vary depending on the type of co-drug employed, e.g. whether it is a steroid or a cyclosporine, on the specific drug employed, on the condition being treated and so forth.

In accordance with the foregoing in a yet further aspect uses as defined above are described herein comprising co-administration, e.g. concomitantly or in sequence, of a therapeutically effective amount of the molecules of the combination of the invention, in free form or in pharmaceutically acceptable salt form, and a second drug substance, said second drug substance being an immunosuppressant, immunomodulatory or anti-inflammatory drug, e.g. as indicated above. Further provided are therapeutic combinations, e.g. a kit, e.g. for use in any method as defined above, comprising the molecules of the invention in free form or in pharmaceutically acceptable salt form, to be used concomitantly or in sequence with at least one pharmaceutical composition comprising an immunosuppressant, immunomodulatory or anti-inflammatory drug.

Gene therapy is currently being used to treat a variety of immune system diseases such as immunoproliferative diseases (e.g. cancers) or autoimmune diseases (e.g. diabetes). The agents of this invention may be administered to a subject by gene therapy in order to regulate transplant rejection and treat immune system diseases. Gene therapy methods include, but are not limited to, *in vivo* methods directly delivering the genes into a subject for in situ transfer into a subject's cells, or *ex vivo* or *in vitro* methods transferring the genes into cells in culture and then transplanting the cultured cells into a subject. Once the gene of interest, encoding one or more of the therapeutic agents, are inserted into cells in a subject, the gene product is expressed and used to ameliorate the disease or symptoms of the disease.

*In vivo,* potential methods for inserting the gene of interest directly into a cell include the usage of recombinant viral vectors such as simian virus 40, adenoviruses, human immunodeficiency virus-1, or other viruses. Methods such as microinjection of the gene of interest directly into cells or other transfer procedures may also be employed.

*Ex vivo* or *in vitro,* potential methods for inserting the gene on interest into cells include transfer procedures such as calcium phosphate mediated transfection, lipid mediated transfection, exposure of the cells to high voltage electric currents to permeabilize the cells (electroporation), microinjection, or encapsulation of the gene of interest into erythrocyte ghosts for transfer into cells.

Gene therapy may be used to treat or augment treatment of various diseases. For example, in the present invention, the DNA encoding one or more of the agents such as soluble CTLA4Ig or soluble gp39 may be inserted into a retroviral vector and packaged into a virus particle. The virus particle is used to infect a subject's cells, transferring the genes of interest into the subject. Once the genes are transferred into a subject, the gene product is expressed in a sufficient amount to regulate transplant rejection and treat immune system diseases.

Further described herein are methods for regulating cell-mediated immune responses and treating diseases in subjects by disrupting the interaction between cell surface molecules with various ligands, thus inducing improved regulation of immune responses and treatment of immune system diseases. Specific interactions that may be disrupted include, but are not limited to, the CTLA4B7, CD28/B7, CD154/CD40 and LFA-1/ICAM pathways. Disruption of three or more interactions leads to enhanced regulation of immune responses, enhanced transplant survival, and enhanced regulation of immunoproliferation.

Also described herein are kits for use in the uses of the invention. Kits may include the three agents of the invention in free form or combined with a pharmaceutically acceptable carrier. Additionally, the kits may also include one or more immunosuppressive agents in conjunction with the three agents of the invention. Potential immunosuppressive agents include, but are not limited to, corticosteroids, nonsteroidal antiinflammatory drugs (e.g. Cox-2 inhibitors), cyclosporin prednisone, azathioprine, methotrexate, TNFα blockers or antagonists, infliximab, any biological agent targeting an inflammatory cytokine, hydroxychloroquine, sulphasalazopryine, gold salts, etanercept, and anakinra.

In addition to the molecules identified herein for the uses of the invention, other molecules (i.e. ligands) can be identified using standard techniques such as binding assays. For example, any of the molecules for use in the invention (e.g. CTLA4, CD28, B7, CD154, CD40, LFA-1, ICAM-1, ICAM-2, ICAM-3, α-actinin, filamin or cytohesin-1 interaction) can be used to screen for ligand molecules including libraries of small molecules in any of a variety of screening techniques. The molecules for use in the invention employed in such screening may be free in solution, affixed to a solid support, or borne on a cell surface. The formation of binding complexes, between any of the molecules of the invention and the agent being tested, may be measured (e.g. published PCT application W084/03564; Price, M. R., et al. 1986; Br. J. Cancer 54:393 (88); Gallegher, G., et al., 1993; Tumour Immunobiology, pages 63-79, Oxford University Press Inc., New York (89)).

Another screening technique involves high throughput screenings of molecules having suitable binding affinity to the molecule of the invention as described in published PCT application W084/03564. In this method, as applied to the molecules for use in the invention, large number of different small test molecules can be synthesized on a solid substrate, such as plastic pins or some other surface. The test molecules can be reacted with the molecules of the invention, and washed. Bound molecules for use in the invention can be then detected by methods well known in the art. Purified molecules for use in the invention can be directly coated on to plates for use in the screening assay. Alternatively, non-neutralizing antibodies (or portions or derivative thereof) can be used to capture the molecule for use in the invention and immobilize it on a solid support.

Another competitive screening assay involves the use of neutralizing antibodies (capable of binding the molecules for use in the invention) to specifically compete with a test molecule for binding to the molecule for use in the invention. In this manner, the antibodies can be used to detect the presence of any protein/peptide molecule which shares one or more antigenic determinants with the molecule for use in the invention.

### EXAMPLE 1: MURINE TAIL SKIN TRANSPLANTS

### Materials and Methods

*Mice.* Adult male 6-8 week old C57BL/6 and BALB/c mice were purchased from Harlan (Indianapolis, Indiana). C57BL/6 mice were transplant donors and BALB/c mice were recipients.

*Reagents.* Murine CTLA4Ig (Linsley (13) and Wallace (87)) and monoclonal antibodies MR1 (hamster anti-murine CD40L/CD154) (56) and M17/5.2 (anti-murine LFA-I/CD11a) have been described previously and were purified from culture supernatants prior to use (62).

*Murine Tail Skin Transplants.* In order to examine skin graft rejection rates in mice, tail skin grafts were studied. Briefly, donor tail skin is removed aseptically from the euthanized donor mice. The tail skin of the recipient is surgically prepped with alcohol and an approximately 1 x 0.5 cm area of recipient skin is removed from the underside of the tail to form a grafting bed. A skin graft from the donor of equal size to the bed is then placed on the bed and covered with a protective glass tube which is fastened to the tail by surgical tape for 2-3 days at which time the tube and tape is removed. Grafts are examined daily for necrosis and are deemed rejected when > 75% necrotic.

The tail skin transplant was performed on day 0. The three agents, CTLA4Ig, MR1 (hamster anti-murine CD40L/CD154) and M17/5.2 (anti-murine LFA-1/CD11a), were administered intraperitoneally, individually or in combinations of two or three agents to the mice. CTLA4Ig was administered in 200 µg dosages on days 0, 2 and 4 post-transplantation. MR1 was administered in 250 µg dosages on days 0, 2 and 4 post-transplantation. Anti-LFA-1 monoclonal antibody was administered in 200 µg dosages on days 0, 1, 2, 3, 4, 5, 6, 14 and 21 post-transplantation.

### Results

### Anti-LFA-1 monoclonal antibody augments lymphocyte interaction blockade inducted graft survival following murine skin transplantation.

Blockade of the LFA-1/ICAM-1 interaction has been previously shown to extend allogeneic skin graft survival in murine models of transplantation. In these models, long-term graft survival is achieved only when a combination of antibodies directed at both LFA-1 and ICAM-1 were administered together. Again, previous studies have demonstrated that soluble CTLA4Ig and anti-CD154 monoclonal antibody MR1, when combined, provide long-term graft survival of allogeneic skin transplants in certain donor/recipient strain combinations. In the BALB/c->C57BL/6 combination, soluble CTLA4Ig and anti-CD154 antibody MR1 when administered alone do not extend graft survival and when administered in combination do not provide long-term graft survival.

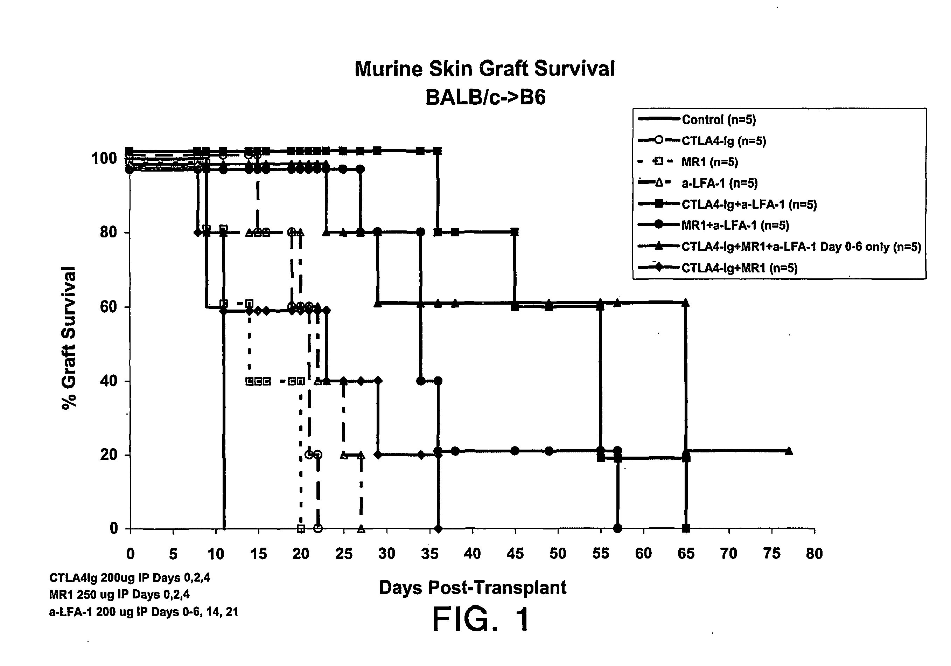

To evaluate the effect of combining anti-LFA-1 monoclonal antibody administration with either soluble CTLA4Ig or anti-CD154 antibody administration, BALB/c donor skin was transplanted onto the tail of C57BL/6 recipients treated with anti-LFA-1 monoclonal antibody M17/5.2, soluble CTLA4Ig, and anti-CD154 monoclonal antibody MR1, or combinations of the three agents. Graft survival was monitored daily and the results are shown in Figure 1. The data demonstrate that administration of anti-LFA-1 along with soluble CTLA4Ig profoundly enhances graft survival when compared to either agent alone. Median graft survival of anti-LFA-1+CTLA4Ig treated mice (55.0 days) was more than either anti-LFA-1 (22.0 days) or soluble CTLA4Ig (21.0 days) alone. Likewise, the data show that combined administration of anti-LFA-1 and anti-CD154 antibody MR1 greatly enhanced graft survival from a median graft survival of 22.0 days and 14.0 days for either anti-LFA-1 or anti-CD154 alone to 34.0 days when administered together. As previously reported, administration of soluble CTLA4Ig and MR1 together does not lead to long-term graft survival (MST=29.0 days) in this skin transplant model; however the addition of anti-LFA-1 monoclonal antibody to this regimen increased median graft survival to 65.0 days. Thus the triple therapy was more successful than double therapy.

### EXAMPLE 2: MURINE NEONATAL HEART TO EAR TRANSPLANTS.

### Materials and Methods

*Mice.* Adult male 6-8 week old C57BL/6 and BALB/c mice were purchased from Harlan (Indianapolis, Indiana). Neonatal C57BL/6 heart donors were bred in our facility. C57BL/6 mice were donors and BALB/c mice were recipients.

*Reagents.* Murine CTLA4-Ig (Linsley (13) and Wallace (84)) and monoclonal antibodies MR1 (hamster anti-murine CD40L/CD154) (56) and M17/5.2 (anti-murine LFA-1/CD11a) have been described previously and were purified from culture supernatants prior to use (62).

*Murine Neonatal Heart to Ear Transplants.* Cardiac transplants were performed as described by *Fulmer et al* (57). Neonatal. hearts are obtained from newborn mice less than 48 hours after birth. The heart is transplanted into recipient mice, which are prepared under anesthesia by making a small incision at the base of the ear pinna and forming a pocket by gently lifting the skin away from the ear. The incision permits the insertion of the donor heart subcutaneously into the pocket. Post-operatively, the cardiac tissue does not begin to beat until 3 to 5 days after transplantation until vascular connections are established. The pulsations of the heart can often be observed under magnification or by measuring contractile activity with an appropriate ECG monitoring device. The mice are observed daily and contractile activity of the transplanted graft monitored daily. The time of graft rejection is defined as the day after transplantation on which contractile activity ceases.

The three agents, QTLA4-Ig, MR1 (hamster anti-murine CD40L/CD154) and M17/5.2 (anti-murine LFA-1/CD11a), were administered intraperitoneally, independently or in combinations of two or three agents to the mice. CTLA4-Ig was administered in 200 µg dosages on days 0, 2 and 4 post-transplantation. MR1 was administered in 250 µg dosages on days 0, 2 and 4 post-transplantation. Anti-LFA-1 monoclonal antibody was administered in 200 µg dosages on days 0, 2, 4 and 6 post-transplantation.

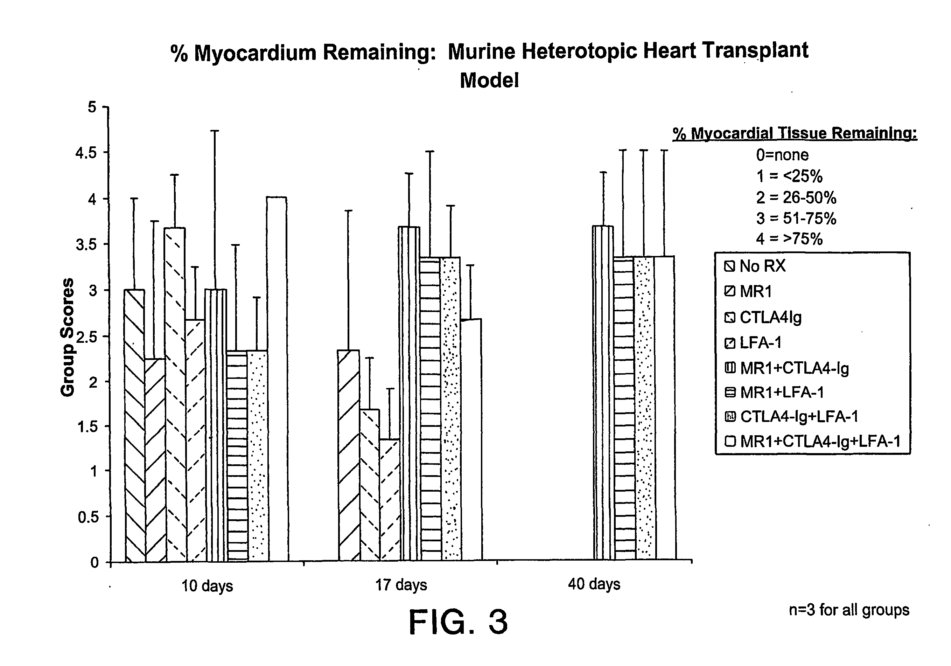

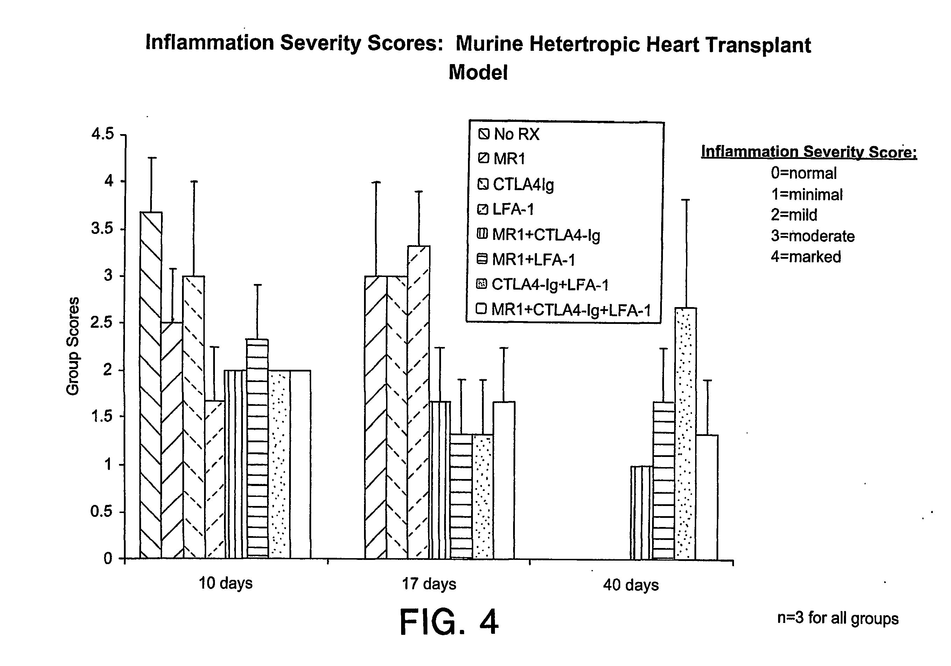

On the days 10, 17 and 40 as indicated in Figures 3 and 4, the mice were euthanized and the grafts were removed and placed in formalin for histopathology. To examine inflammatory infiltration and myocardium damage, hematoxylin and eosin (H&E) staining on cut sections was performed. The results of the myocardium damage analysis are shown in Figure 3 with the figure indicating the percent cardiac tissue remaining in the graft for each group i.e. higher scores indicate a greater percentage of cardiac tissue present and, accordingly, less tissue damage. The results of the inflammatory infiltration are shown in Figure 4 with the lower scores indicating less inflammation present in the graft for the various treatment groups.

### Results

### Anti-LFA-1 monoclonal antibody augments lymphocyte Interaction blockade induced graft survival following murine cardiac transplantation.

Blockade of the LFA-1/ICAM-1 interaction has been previously shown to extend allogeneic cardiac graft survival in murine models of transplantation. In these models, long-term graft survival is achieved only when a combination of antibodies directed at both LFA-1 and ICAM-1 were administered together. Likewise, previous studies have demonstrated that soluble CTLA4-Ig and anti-CD154 antibody when combined, provide long-term graft survival of allogeneic heart transplants. Alone, each agent extends graft survival although significantly less then when administered together.

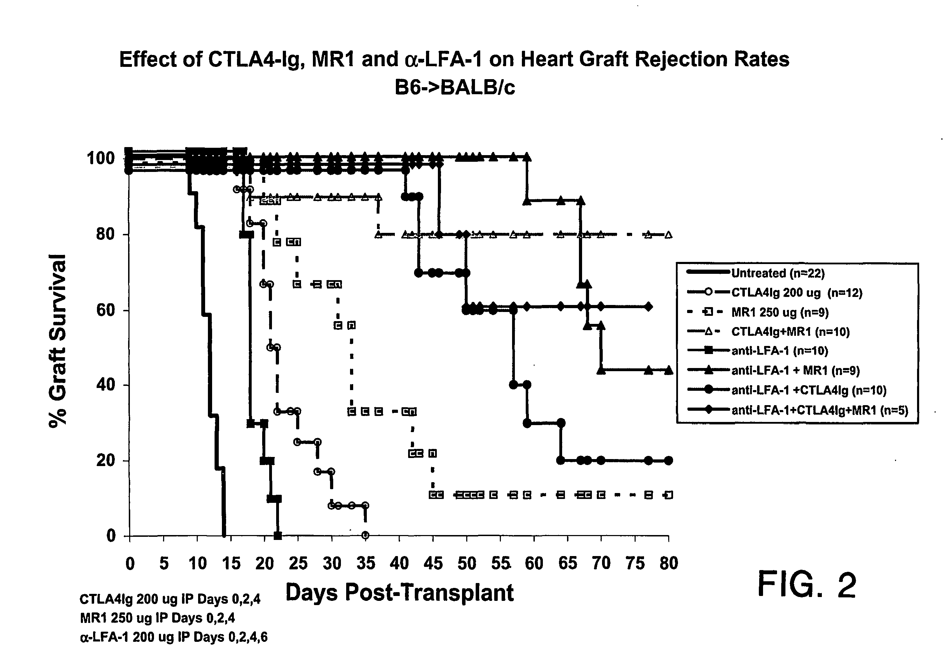

To evaluate the effect of combining anti-LFA-1 administration with either soluble CTLA4-Ig or anti-CD154 administration, neonatal C57BL/6 hearts were subcutaneously implanted into the ear pinna of adult BALB/c recipients treated with anti-LFA-1 monoclonal antibody, soluble CTLA4-Ig, anti-CD154 monoclonal antibody, or combinations of the three agents. Contractile activity of these hearts were monitored daily by ECG and the heart graft survival results are shown in Figure 2. The data demonstrate that administration of anti-LFA-1 along with soluble CTLA4-Ig profoundly enhances graft survival when compared to either agent alone. Median graft survival of anti-LFA-1+CTLA4-Ig treated mice (57 days) was more than double either anti-LFA-1 (18 days) or soluble CTLA4-Ig (20 days) alone (p<0.0003). Likewise, the data show that combined administration of anti-LFA-1 and anti-CD154 greatly enhanced graft survival from a median graft survival of 18.0 days and 33.0 days for either anti-LFA-1 or anti-CD154 alone to 67.5 days when administered together. As previously reported, administration of soluble CTLA4-Ig and anti-CD40L leads to very long-term graft survival (MST>80 days).

The data results further indicate that the groups that received multiple therapies (CTLA4Ig, MR1 and anti-LFA-1 either in double or triple combinations), retained a higher percentage of myocardial tissue (Figure 3) and had less inflammation (Figure 4) than the other therapy groups.

### EXAMPLE 3: CONSTRUCTION OF HUMAN CTLA4Ig AND L104EA29YIg

This example provides a description of the methods used to generate the nucleotide sequences encoding the human soluble CTLA4 molecules e.g. CTLA4Ig, L104EA29YIg. A nucleotide sequence encoding CTLA4Ig was first generated as described *infra,* then a single-site mutant L104EIg was derived from the CTLA4Ig sequence and tested for binding kinetics for CD80 and/or CD86. The L104EIg nucleotide sequence was used as a template to generate the double-site mutant CTLA4 sequence, L104EA29YIg, which was tested for binding kinetics for CD80 and/or CD86.

### Construction of CTLA4Ig

A genetic construct encoding CTLA41g comprising the extracellular domain of CTLA4 and an IgCgammal domain was constructed as described in U. S. Patents 5,844,095 and 5,851,795. The extracellular domain of the CTLA4 gene was cloned by PCR using synthetic oligonucleotides corresponding to the published sequence as described by Dariavach et al. (72).

Because a signal peptide for CTLA4 was not identified in the CTLA4 gene, the N-terminus of the predicted sequence of CTLA4 was fused to the signal peptide of oncostatin M (73) in two steps using overlapping oligonucleotides. For the first step, the oligonucleotide, CTCAGTCTGGTCCTTGCACTCCTGTTTCCAAGCATGGCGAGCA TGGCAATGCACGTGGCCCAGCC (which encoded the C terminal 15 amino acids from the oncostatin M signal peptide fused to the N terminal 7 amino acids of CTLA4) was used as forward primer, and TTTGGGCTCCTGATCAGAATCTGGGCACGGTTG (encoding amino acid residues 119-125 of the amino acid sequence encoding CTLA4 receptor and containing a Bcl I restriction enzyme site) as reverse primer. The template for this step was cDNA synthesized from 1 micro g of total RNA from H38 cells (an HTLV II infected T-cell leukemic cell line provided by Drs. Salahudin and Gallo, NCI, Bethesda, MD). A portion of the PCR product from the first step was reamplified, using an overlapping forward primer, encoding the N terminal portion of the oncostatin M signal peptide and containing a Hind III restriction endonuclease site, CTAGCCACTGAAGCTTCACCAATGGGTGTACTGCTCACACAGAGGACGCTGC TCAGTCTGGTCCTTGCACTC and the same reverse primer. The product of the PCR reaction was digested with Hind III and Bcl I and ligated together with a Bcl I/Xba I cleaved cDNA fragment encoding the amino acid sequences corresponding to the hinge, CH2 and CH3 regions of IgC1 into the Hind III/Xba I cleaved expression vector, CDM8 or Hind III/Xba I cleaved expression vector piLN (also known as πLN).

DNA encoding the amino acid sequence corresponding to CTLA4Ig has been deposited with the ATCC under the Budapest Treaty on May 31, 1991, and has been accorded ATCC accession number 68629.

### CTLA4Ig Codon Based Mutagenesis to generate double mutants:

A mutagenesis and screening strategy was developed to identify mutant CTLA4Ig molecules that had slower rates of dissociation ("off" rates) from CD80 and/or CD86 molecules. Single-site mutant nucleotide sequences were generated using CTLA4Ig (U.S. Patent Numbers: 5,844,095; 5,851,795; and 5,885,796; ATCC Accession No. 68629) as a template. Mutagenic oligonucleotide PCR primers were designed for random mutagenesis of a specific cDNA codon by allowing any base at positions 1 and 2 of the codon, but only guanine or thymine at position 3 (XXG/T; also known as NNG/T). In this manner, a specific codon encoding an amino acid could be randomly mutated to code for each of the 20 amino acids. In that regard, XXG/T mutagenesis yields 32 potential codons encoding each of the 20 amino acids. PCR products encoding mutations in close proximity to -M97-G107 of CTLA4Ig (see Figure 5), were digested with SacI/XbaI and subcloned into similarly cut CTLA4Ig πLN expression vector. This method was used to generate the single-site CTLA4 mutant molecule L104EIg.

For mutagenesis in proximity to S25-R33 of CTLA4Ig, a silent NheI restriction site was first introduced 5' to this loop, by PCR primer-directed mutagenesis. PCR products were digested with NheI/XbaI and subcloned into similarly cut CTLA4Ig or L104EIg expression vectors. This method was used to generate the double-site CTLA4 mutant molecule L104EA29YIg (Figure 6). In particular, the nucleic acid molecule encoding the single-site CTLA4 mutant molecule, L104EIg, was used as a template to generate the double-site CTLA4 mutant molecule, L104EA29YIg. The sequence of L104EA29YIg is shown in Figure 6 and includes an N-terminal leader sequence.

The following provides a description of the screening methods used to identify the single-and double-site mutant CTLA4 polypeptides, expressed from the constructs described *supra,* that exhibited a higher binding avidity for CD80 and CD86 antigens, compared to non-mutated CTLA4Ig molecules.

Current *in vitro* and *in vivo* studies indicate that CTLA4Ig by itself is enable to completely block the priming of antigen specific activated T cells. *In vitro* studies with CTLA4Ig and either monoclonal antibody specific for CD80 or CD86 measuring inhibition of T cell proliferation indicate that anti-CD80 monoclonal antibody did not augment CTLA4Ig inhibition. However, anti-CD86 monoclonal antibody did augment the inhibition, indicating that CTLA4Ig was not as effective at blocking CD86 interactions. These data support earlier findings by Linsley et al. (74) showing inhibition of CD80-mediated cellular responses required approximately 100 fold lower CTLA4Ig concentrations than for CD86-mediated responses. Based on these findings, it was surmised that soluble CTLA4 mutant molecules having a higher avidity for CD86 than wild type CTLA4 should be better able to block the priming of antigen specific activated cells than CTLA4Ig.

To this end, the soluble CTLA4 mutant molecules described above were screened using a novel screening procedure to identify several mutations in the extracellular domain of CTLA4 that improve binding avidity for CD80 and CD86. This screening strategy provided an effective method to directly identify mutants with apparently slower "off" rates without the need for protein purification or quantitation since "off" rate determination is concentration independent as described by O'Shannessy et al (75).

COS cells were transected with individual miniprep purified plasmid DNA and propagated for several days. Three day conditioned culture media was applied to BIAcore biosensor chips (Pharmacia Biotech AB, Uppsala, Sweden) coated with soluble CD80Ig or CD86Ig. The specific binding and dissociation of mutant proteins was measured.by surface plasmon resonance (75). All experiments were run on BIAcore™ or BIAcore™ 2000 biosensors at 25°C. Ligands were immobilized on research grade NCM5 sensor chips (Pharmacia) using standard N-ethyl-N'-(dimethylaminopropyl) carbodiimidN-hydroxysuccinimide coupling (76, 77).

### Screening Method

COS cells grown in 24 well tissue culture plates were transiently transfected with DNA encoding mutant CTLA4Ig. Culture media containing secreted soluble mutant CTLA4Ig was collected 3 days later.

Conditioned COS cell culture media was allowed to flow over BIAcore biosensor chips derivatized with CD86Ig or CD80Ig as described in Greene et al. (78), and mutant molecules were identified with "off" rates slower than that observed for wild type CTLA4Ig. The cDNAs corresponding to selected media samples were sequenced and DNA was prepared to perform larger scale COS cell transient transfection, from which mutant CTLA4Ig protein was prepared following protein A purification of culture media.

BIAcore analysis conditions and equilibrium binding data analysis were performed as described in Greene et al. (78).

### BIAcore Data Analysis

Senosorgram baselines were normalized to zero response units (RU) prior to analysis. Samples were run over mock-derivatized flow cells to determine background response unit (RU) values due to bulk refractive index differences between solutions. Equilibrium dissociation constants (K_{d}) were calculated from plots of R_{eq} versus C, where R_{eq} is the steady-state response minus the response on a mock-derivatized chip, and C is the molar concentration of analyte. Binding curves were analyzed using commercial nonlinear curve-fitting software (Prism, GraphPAD Software).

Experimental data were first fit to a model for a single ligand binding to a single receptor (1-site model, i.e., a simple langmuir system, A+B↔AB), and equilibrium association constants (K_{d}=[A]•[B]\[AB]) were calculated from the equation R=Rₘₐₓ•C/(K_{d}+C). Subsequently, data were fit to the simplest two-site model of ligand binding (i.e., to a receptor having two non-interacting independent binding sites as described by the equation R=Rₘₐₓ•C\(K_{d1}+C)+Rₘₐₓ₂•C\(K_{d2}+C)).

The goodness-of-fits of these two models were analyzed visually by comparison with experimental data and statistically by an F test of the sums-of-squares. The simpler one-site model was chosen as the best fit, unless the two-site model fit significantly better (p<0.1).

Association and disassociation analyses were performed using BIA evaluation 2.1 Software (Pharmacia). Association rate constants kₒₙ were calculated in two ways, assuming both homogenous single-site interactions and parallel two-site interactions. For single-site interactions, kₒₙ values were calculated according to the equation Rₜ=R_{eq}(1-exp^{-ks(t-t}₀), where Rₜ is a response at a given time, t; R_{eq} is the steady-state response; to is the time at the start of the injection; and kₛ=dR/dt=kₒₙ•Ck_{off}, and where C is a concentration of analyte, calculated in terms of monomeric binding sites.. For two-site interactions kₒₙ values were calculated according to the equation Rₜ=R_{eq1}(1-exp^{-ks1(t-t}₀⁾+R_{eq2}(1-exp^{ks2(t-t}₀⁾. For each model, the values of kₒₙ were determined from the calculated slope (to about 70% maximal association) of plots of kₛ versus C.

Dissociation data were analyzed according to one site (AB=A+B) or two sites (AiBj=Ai+Bj) models, and rate constants (k_{off}) were calculated from best fit curves. The binding site model was used except when the residuals were greater than machine background (2-10 RU, according to machine), in which case the two-binding site model was employed. Half-times of receptor occupancy were calculated using the relationship t_{1/2}=0.693/k_{off}.

### Flow Cytometry:

Murine mAb L307.4 (anti-CD80) was purchased from Becton Dickinson (San Jose, California) and IT2.2 (anti-B7-0 [also known as CD86]), from Pharmingen (San Diego, California). For immunostaining, CD80-positive and/or CD86-positive CHO cells were removed from their culture vessels by incubation in phosphate-buffered saline (PBS) containing 10mM EDTA. CHO cells (1-10 x 10⁵) were first incubated with mAbs or immunoglobulin fusion proteins in DMEM containing 10% fetal bovine serum (FBS), then washed and incubated with fluorescein isothiocyanate-conjugated goat anti-mouse or anti-human immunoglobulin second step reagents (Tago, Burlingame, California). Cells were given a final wash and analyzed on a FACScan (Becton Dickinson).

### SDS-PAGE and Size Exclusion Chromatography

SDS-PAGE was performed on Tris/glycine 4-20% acrylamide gels (Novex, San Diego, CA). Analytical gels were stained with Coomassie Blue, and images of wet gels were obtained by digital scanning. CTLA4Ig (25 µg) and L104EA29YIg (25 µg) were analyzed by size exclusion chromatography using a TSK-GEL G300 SW_{XL} column (7.8 x 300mm, Tosohaas, Montgomeryville, PA) equilibrated in phosphate buffered saline containing 0.02% NAN₃ at a flow rate of 1.0 ml/min.

### CTLA4X_{C120S} and L104EA29YX_{C120S.}

Single chain CTLA4X_{C120S} was prepared as previously described (Linsley et al., (1995) J. Biol. Chem., 270:15417-15424 (84)). Briefly, an oncostatin M CTLA4 (OMCTLA4) expression plasmid was used as a template, the forward primer, GAGGTGATAAAGCTTCACCAATGGGTGTACTGCTCACACAG was chosen to match sequences in the vector; and the reverse primer, GTGGTGTATTGGTCTAGATCAATCAGAATCTGGGCACGGTTC corresponded to the last seven amino acids (i.e. amino acids 118-124) in the extracellular domain of CTLA4, and contained a restriction enzyme site, and a stop codon (TGA). The reverse primer specified a C120S (cysteine to serine at position 120) mutation. In particular, the nucleotide sequence GCA (nucleotides 34-36) of the reverse primer shown above is replaced with one of the following nucleotide sequences: AGA, GGA, TGA, CGA, ACT, or GCT. As persons skilled in the art will understand, the nucleotide sequence GCA is a reversed complementary sequence of the codon TGC for cysteine. Similarly, the nucleotide sequences AGA, GGA, TGA, CGA, ACT, or GCT are the reversed complementary sequences of the codons for serine. Polymerase chain reaction products were digested with *Hind*III/*Xba*I and directionally subcloned into the expression vector πLN (Bristol-Myers Squibb Company, Princeton, NJ). L104EA29YX_{C120S} was prepared in an identical manner. Each construct was verified by DNA sequencing.

### Identification and Biochemical Characterization of High Avidity Mutants

Twenty four amino acids were chosen for mutagenesis and the resulting ~2300 mutant proteins assayed for CD86Ig binding by surface plasmon resonance (SPR; as described, supra). The predominant effects of mutagenesis at each site are summarized in Table II. Random mutagenesis of some amino acids in the S25-R33 apparently did not alter ligand binding. Mutagenesis of E31 and R33 and residues M97-Y102 apparently resulted in reduced ligand binding. Mutagenesis of residues, S25, A29, and T30, K93, L96, Y103, L104, and G105, resulted in proteins with slow "on" and/or slow "off' rates. These results confirm previous findings that residues in the S25-R33 region, and residues in or near M97-Y102 influence ligand binding (Peach et al:, (1994) J. Exp. Med., 180:2049-2058(85)).

Mutagenesis of sites S25, T30, K93, L96, Y103, and G105 resulted in the identification of some mutant proteins that had slower "off" rates from CD86Ig. However, in these instances, the slow "off' rate was compromised by a slow "on" rate which resulted in mutant proteins with an overall avidity for CD86Ig that was apparently similar to that seen with wild type CTLA4Ig. In addition, mutagenesis of K93 resulted in significant aggregation which may have been responsible for the kinetic changes observed.
Random mutagenesis of L104 followed by COS cell transfection and screening by SPR of culture media samples over immobilized CD86Ig yielded six media samples containing mutant proteins with approximately 2-fold slower "off" rates than wild type CTLA4Ig. When the corresponding cDNA of these mutants were sequenced, each was found to encode a leucine to glutamic acid mutation (L104E). Apparently, substitution of leucine 104 to aspartic acid (L104D) did not affect CD86Ig binding.

Mutagenesis was then repeated at each site listed in Table II, this time using L104E as the PCR template instead of wild type CTLA4Ig, as described above. SPR analysis, again using immobilized CD86Ig, identified six culture media samples from mutagenesis of alanine 29 with proteins having approximately 4-fold slower "off" rates than wild type CTLA4Ig. The two slowest were tyrosine substitutions (L104EA29Y), two were leucine (L104EA29L), one was tryptophan (L104EA29W), and one was threonine (L104EA29T). Apparently, no slow "off" rate mutants were identified when alanine 29 was randomly mutated, alone, in wild type CTLA4Ig.

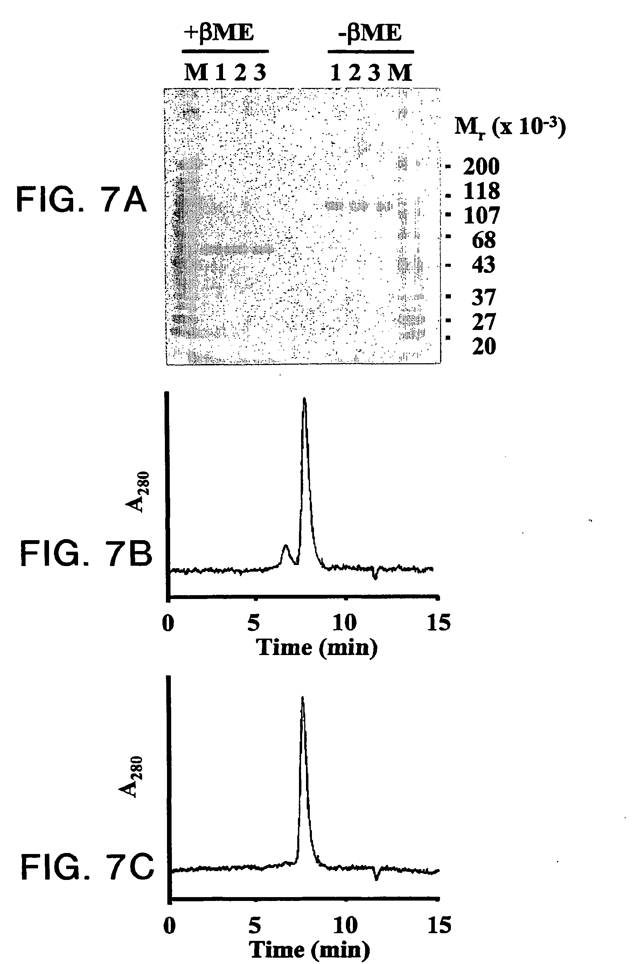

The relative molecular mass and state of aggregation of purified L104E and L104EA29YIg was assessed by SDS-PAGE and size exclusion chromatography. L104EA29YIg (~1 µg; lane 3) and L104EIg (~1 µg; lane 2) apparently had the same electrophoretic mobility as CTLA4Ig (~1 µg; lane 1) under reducing (~50kDa; +BME; plus 2-mercaptoethanol) and non-reducing (~100kDa; -BME) conditions (FIG. 7A). Size exclusion chromatography demonstrated that L104EA29YIg (FIG. 7C) apparently had the same mobility as dimeric CTLA4Ig (FIG. 7B). The major peaks represent protein dimer while the faster eluting minor peak in FIG. 7B represents higher molecular weight aggregates. Approximately 5.0% of CTLA4Ig was present as higher molecular weight aggregates but there was no evidence of aggregation of L104EA29YIg or L104EIg. Therefore, the stronger binding to CD86Ig seen with L104EIg and L104EA29YIg could not be attributed to aggregation induced by mutagenesis.

### Equilibrium and Kinetic Binding Analysis

Equilibrium and kinetic binding analysis was performed on protein A purified CTLA4Ig, L104EIg, and L104EA29YIg using surface plasmon resonance (SPR). The results are shown in Table I. Observed equilibrium dissociation constants (K_{d}; Table I) were calculated from binding curves generated over a range of concentrations (5.0-200 nM). L104EA29YIg binds more strongly to CD86Ig than does L104EIg or CTLA4Ig. The lower K_{d} of L104EA29YIg (3.21 nM) than L104EIg (6.06 nM) or CTLA4Ig (13.9 nM) indicates higher binding avidity of L104EA29YIg to CD86Ig. The lower K_{d} of L104EA29YIg (3.66 nM) than L104EIg (4.47 nM) or CTLA4Ig (6.51 nM) indicates higher binding avidity of L104EA29YIg to CD80Ig.

Kinetic binding analysis revealed that the comparative "on" rates for CTLA4Ig, L104EIg, and L104EA29YIg binding to CD80 were similar, as were the "on" rates for CD86Ig (Table I). However, "off" rates for these molecules were not equivalent (Table I). Compared to CTLA4Ig, L104EA29YIg had approximately 2-fold slower "off' rate from CD80Ig, and approximately 4-fold slower "off" rate from CD86Ig. L104E had "off' rates intermediate between L104EA29YIg and CTLA4Ig. Since the introduction of these mutations did not significantly affect "on" rates, the increase in avidity for CD80Ig and CD86Ig observed with L104EA29YIg was likely primarily due to a decrease in "off" rates.

To determine whether the increase in avidity of L104EA29YIg for CD86Ig and CD80Ig was due to the mutations affecting the way each monomer associated as a dimer, or whether there were avidity enhancing structural changes introduced into each monomer, single chain constructs of CTLA4 and L104EA29Y extracellular domains were prepared following mutagenesis of cysteine 120 to serine as described supra, and by Linsley et al., (1995) J. Biol. Chem., 270:15417-15424 (84). The purified proteins CTLA4X_{C120S} and L104EA29YX_{C120S} were shown to be monomeric by gel permeation chromatography (Linsley et al., (1995), supra (84)), before their ligand binding properties were analyzed by SPR. Results showed that binding affinity of both monomeric proteins for CD86Ig was approximately 35-80-fold less than that seen for their respective. dimers (Table I). This supports previously published data establishing that dimerization of CTLA4 was required for high avidity ligand binding (Greene et al., (1996) J. Biol. Chem., 271:26762-26771 (78)).

L104EA29YX_{C120S} bound with approximately 2-fold higher affinity than CTLA4X_{C120S} to both CD80Ig and CD86Ig. The increased affinity was due to approximately 3-fold slower rate of dissociation from both ligands. Therefore, stronger ligand binding by L104EA29Y was most likely due to avidity enhancing structural changes that had been introduced into each monomeric chain rather than alterations in which the molecule dimerized.

### Location and Structural Analysis of Avidity Enhancing Mutations

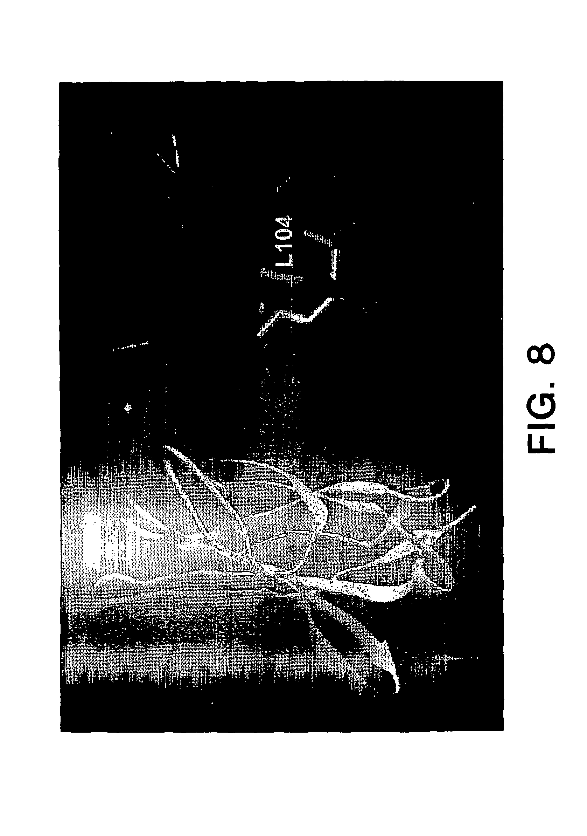

The solution structure of the extracellular IgV-like domain of CTLA4 has recently been determined by NMR spectroscopy (Metzler et al., (1997) Nature Struct. Biol., 4:527-531 (86)). This allowed accurate location of leucine 104 and alanine 29 in the three dimensional fold (FIG. 8A-B). Leucine 104 is situated near the highly conserved MYPPPY amino acid sequence. Alanine 29 is situated near the C-terminal end of the S25-R33 region, which is spatially adjacent to. the MYPPPY region. While there is significant interaction between residues at the base of these two regions, there is apparently no direct interaction between L104 and A29 although they both comprise part of a contiguous hydrophobic core in the protein. The structural consequences of the two avidity enhancing mutants were assessed by modeling. The A29Y mutation can be easily accommodated in the cleft between the S25-R33 region and the MYPPPY region, and may serve to stabilize the conformation of the MYPPPY region. In wild type CTLA4, L104 forms extensive hydrophobic interactions with L96 and V94 near the MYPPPY region. It is highly unlikely that the glutamic acid mutation adopts a conformation similar to that of L104 for two reasons. First, there is insufficient space to accommodate the longer glutamic acid side chain in the structure without significant perturbation to the S25-R33 region. Second, the energetic costs of burying the negative charge of the glutamic acid side chain in the hydrophobic region would be large. Instead, modeling studies predict that the glutamic acid side chain flips out on to the surface where its charge can be stabilized by solvation. Such a conformational change can easily be accommodated by G105, with minimal distortion to other residues in the regions.

### Binding of High Avidity Mutants to CHO Cells Expressing CD80 or CD86

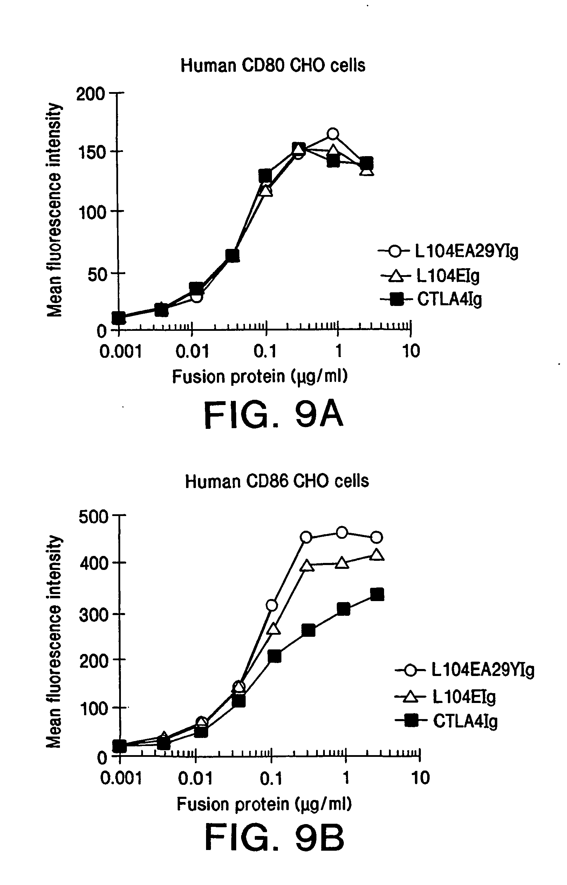

FACS analysis (Fig. 9) of CTLA4Ig and mutant molecules binding to stably transfected CD80+ and CD86+CHO cells was performed as described herein. CD80-positive and CD86-positive CHO cells were incubated with increasing concentrations of CTLA4Ig, L104EA29YIg, or L104EIg, and then washed. Bound immunoglobulin fusion protein was detected using fluorescein isothiocyanate-conjugated goat anti-human immunoglobulin.

As shown in Figure 9, CD80-positive or CD86-positive CHO cells (1.5x10⁵) were incubated with the indicated concentrations of CTLA4Ig (closed squares), L104EA29YIg (circles), or L104EIg (triangles) for 2 hr. at 23°C, washed, and incubated with fluorescein isothiocyanate-conjugated goat anti-human immunoglobulin antibody. Binding on a total of 5,000 viable cells was analyzed (single determination) on a FACScan, and mean fluorescence intensity (MFI) was determined from data histograms using PC-LYSYS. Data were corrected for background fluorescence measured on cells incubated with second step reagent only (MFI= 7). Control L6 mAb (80 µg/ml) gave MFI < 30. These results are representative of four independent experiments.

Binding of L104EA29YIg, L104EIg, and CTLA4Ig to human CD80-transfected CHO cells is approximately equivalent (FIG. 9A). L104EA29YIg and L104EIg bind more strongly to CHO cells stably transfected with human CD86 than does CTLA4Ig (FIG. 9B).

### Functional Assays:

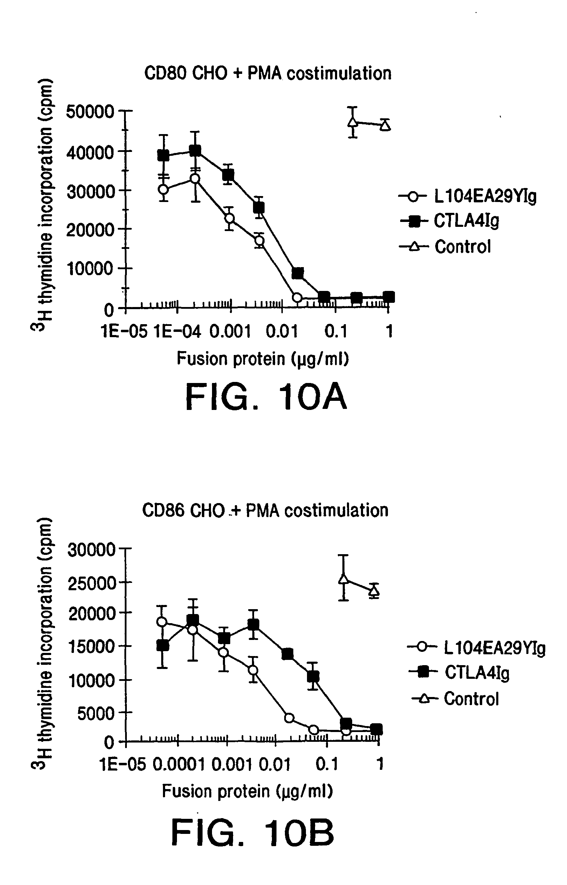

Human CD4-positive T cells were isolated by immunomagnetic negative selection (Linsley et al., (1992) J. Exp. Med. 176:1595-1604 (83)). Isolated CD4-positive T cells were stimulated with phorbal myristate acetate (PMA) plus CD80-positive or CD86-positive CHO cells in the presence of titrating concentrations of inhibitor. CD4-positive T cells (8-10 x 10⁴/well) were cultured in the presence of 1 nM PMA with or without irradiated CHO cell stimulators. Proliferative responses were measured by the addition of 1 µCi/well of [3H]thymidine during the final 7 hours of a 72 hour culture. Inhibition of PMA plus CD80-positive CHO, or CD86-positive CHO, stimulated T cells by L104EA29YIg and CTLA4Ig was performed. The results are shown in FIG. 10. L104EA29YIg inhibits proliferation of CD80-positive PMA treated CHO cells more than CTLA4Ig (FIG. 10A). L104EA29YIg is also more effective than CTLA4Ig at inhibiting proliferation of CD86-positive PMA treated CHO cells (FIG. 10B). Therefore, L104EA29YIg is a more potent inhibitor of both CD80- and CD86-mediated costimulation of T cells.

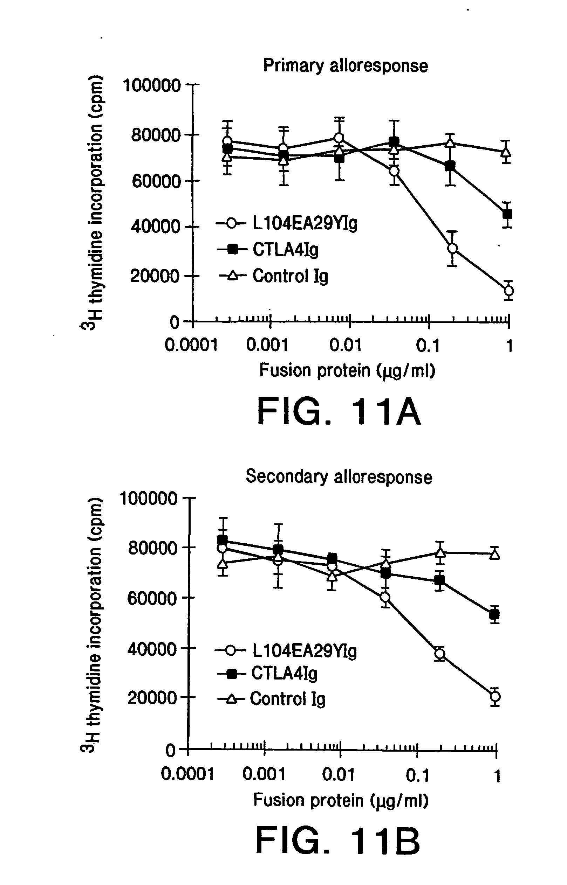

Figure 11 shows inhibition by L104EA29YIg and CTLA4Ig of allostimulated human T cells prepared above, and further allostimulated with a human B lymphoblastoid cell line (LCL) called PM that expressed CD80 and CD86 (T cells at 3.0x10⁴/well and PM at 8.0x10³/well). Primary allostimulation occurred for 6 days, then the cells were pulsed with ³H-thymidine for 7 hours, before incorporation of radiolabel was determined.

Secondary allostimulation was performed as follows. Seven day primary allostimulated T cells were harvested over lymphocyte separation medium (LSM) (ICN, Aurora, OH) and rested for 24 hours. T cells were then restimulated (secondary), in the presence of titrating amounts of CTLA4Ig or L104EA29YIg, by adding PM in the same ratio as above. Stimulation occurred for 3 days, then the cells were pulsed with radiolabel and harvested as above. The effect of L104EA29YIg on primary allostimulated T cells is shown in FIG. 11A. The effect of L 104EA29YIg on secondary allostimulated T cells is shown in FIG. 11B. L104EA29YIg inhibits both primary and secondary T cell proliferative responses better than CTLA4Ig.

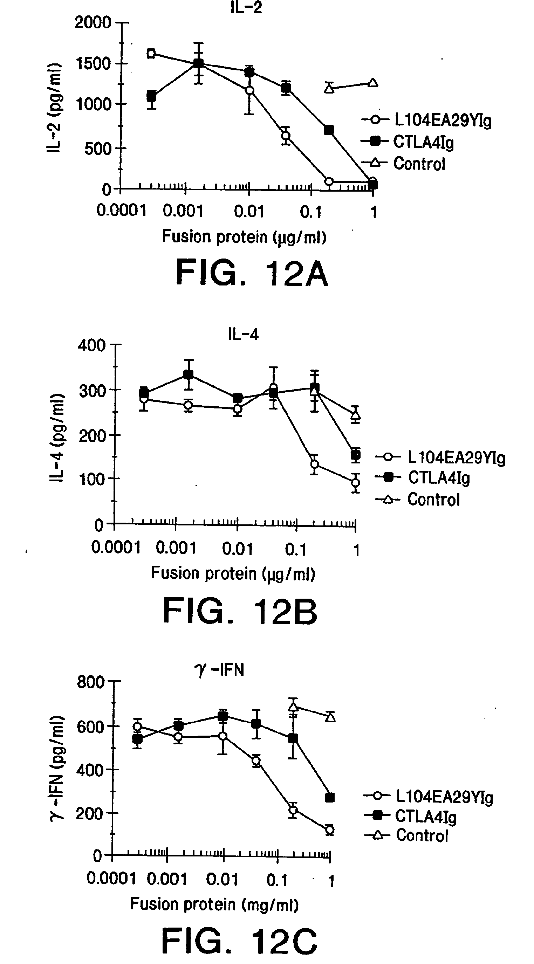

To measure cytokine production (Figure 12), duplicate secondary allostimulation plates were set up. After 3 days, culture media was assayed using ELISA kits (Biosource, Camarillo, CA) using conditions recommended by the manufacturer. L104EA29YIg was found to be more potent than CTLA4Ig at blocking T cell IL-2, IL-4, and γ-IFN cytokine production following a secondary allogeneic stimulus (FIGS. 12A-C).

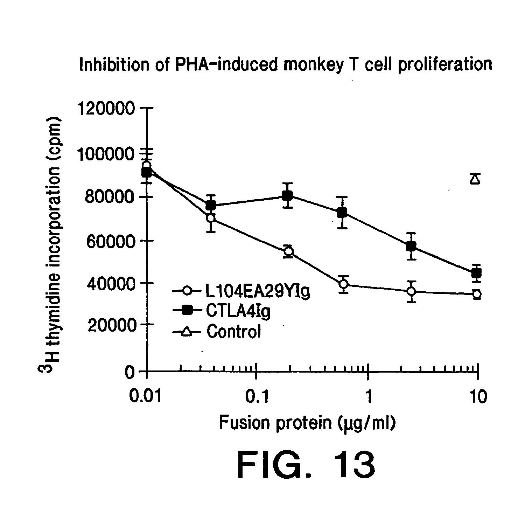

The effects of L104EA29YIg and CTLA4Ig on monkey mixed lymphocyte response (MLR) are shown in Figure 13. Peripheral blood mononuclear cells (PBMC'S; 3.5x10⁴ cells/well from each monkey) from 2 monkeys were purified over lymphocyte separation medium (LSM) and mixed with 2µg/ml phytohemaglutinin (PHA). The cells were stimulated 3 days then pulsed with radiolabel 16 hours before harvesting. L104EA29YIg inhibited monkey T cell proliferation better than CTLA4Ig.

**Table I:**

| Equilibrium and apparent kinetic constants are given in the following table (values are means ± standard deviation from three different experiments): | | | | |
|---|---|---|---|---|
| **Immobilized Protein** | **Analyte** | ***k*ₒₙ (x 10⁵) *M*⁻*¹ S*^{-*1*}** | ***k*_{off} (x 10⁻³) *S*^{-*1*}** | ***K*_{d} *nM*** |
| CD80Ig | CTLA4Ig | 3.44 ± 0.29 | 2.21 ± 0.18 | 6.51 ± 1.08 |
| CD80Ig | L104EIg | 3.02 ±0.05 | 1.35 ± 0.08 | 4.47 ± 0.36 |
| CD80Ig | L104EA29YIg | 2.96 ± 0.20 | 1.08 ± 0.05 | 3.66 ± 0.41 |
| CD80Ig | CTLA4X_{C120S} | 12.0 ± 1.0 | 230 ± 10 | 195 ± 25 |
| CD80Ig | L104EA29YX_{C120S} | 8.3 ± 0.26 | 71 ± 5 | 85.0 ± 2.5 |
| | | | | |
| CD86Ig | CTLA4Ig | 5.95 ± 0.57 | 8.16 ± 0.52 | 13.9 ± 2.27 |
| CD86Ig | L104EIg | 7.03 ± 0.22 | 4.26 ± 0.11 | 6.06 ± 0.05 |
| CD86Ig | L104EA29YIg | 6.42 ± 0.40 | 2.06 ± 0.03 | 3.21 ± 0.23 |
| CD86Ig | CTLA4X_{C120S} | 16.5 ± 0.5 | 840 ± 55 | 511 ± 17 |
| CD86Ig | L104EA29YX_{C120S} | 11.4 ± 1.6 | 300 ± 10 | 267 ± 29 |

**Table II**

| The effect on CD86Ig binding by mutagenesis of CTLA4Ig at the sites listed was determined by SPR, described supra. The predominant effect is indicated with a "+" sign. | | | |
|---|---|---|---|
| Mutagenesis Site | Effects of Mutagenesis No Apparent Effect | Slow "on" rate/ slow "off rate | Reduced ligand binding |
| S25 | | + | |
| P26 | + | | |
| G27 | + | | |
| K28 | + | | |
| A29 | | + | |
| T30 | | + | |
| E31 | | | + |
| R33 | | | + |
| K93 | | + | |
| L96 | | + | |
| M97 | | | + |
| Y98 | | | + |
| P99 | | | + |
| P100 | | | + |
| P101 | | | + |
| Y102 | | | + |
| Y103 | | + | |
| L104 | | + | |
| G105 | | + | |
| I106 | + | | |
| G107 | + | | |
| Q111 | + | | |
| Y113 | + | | |
| I115 | + | | |

### REFERENCES

1. Greenfield, E.A., K.A. Nguyen, and V.K. Kuchroo. 1998. CD28/B7 costimulation: a review. Crit.Rev.Immunol. 18:389.
2. Aruffo, A. and B. Seed. 1987. Molecular cloning of a CD28 cDNA by a high-efficiency COS cell expression system. Proc.Natl.Acad.Sci. U.S.A. 84:8573.
3. Linsley, P.S. and J.A. Ledbetter. 1993. The role of the CD28 receptor during T cell responses to antigen. Annu.Rev.Immunol. 11:191.
4. June, C.H., J.A. Bluestone, L.M. Nadler, and C.B. Thompson. 1994. The B7 and CD28 receptor families. Immunol. Today 15:321.
5. Lenschow, D.J., T.L. Walunas, and J.A. Bluestone. 1996. CD28/B7 system of T cell costimulation. Annu.Rev.Immunol. 14:233.
6. Van-Gool, S.W., P. Vandenberghe, M. de-Boer, and J.L. Ceuppens. 1996. CD80, CD86 and CD40 provide accessory signals in a multiple-step T-cell activation model. Immunol.Rev. 153:47*.*
7. Hollenbaugh, D., L.S. Grosmaire, C.D. Kullas, N.J. Chalupny, A.S. Braesch, R.J. Noelle, I. Stamenkovic, J.A. Ledbetter, and A. Aruffo. 1992. The human T cell antigen gp39, a member of the TNF gene family, is a ligand for the CD40 receptor: expression of a soluble form of gp39 with B cell co-stimulatory activity. EMBO J 11:4313.
8. Graf, D., U. Korthauer, H.W. Mages, G. Senger, and R.A. Kroczek. 1992. Cloning of TRAP, a ligand for CD40 on human T cells. Eur.J.Immunol. 22:3191.
9. Armitage, R.J., W.C. Fanslow, L. Strockbine, T.A. Sato, K.N. Clifford, B.M. Macduff, D.M. Anderson, S.D. Gimpel, S.T. Davis, C.R. Maliszewski, and a. et. 1992. Molecular and biological characterization of a murine ligand for CD40. Nature 357:80.
10. Daikh, D., D. Wofsy, and J.B. Imboden. 1997. The CD28-B7 costimulatory pathway and its role in autoimmune disease. J Leukoc. Biol. 62:156.
11. Tan, R., S.J. Teh, J.A. Ledbetter, P.S. Linsley, and H.S. Teh. 1992. B7 costimulates proliferation of CD4-8+ T lymphocytes but is not required for the deletion of immature CD4+8+ thymocytes. J.Immunol. 149:3217.
12. Linsley, P.S., P.M. Wallace, J. Johnson, M.G. Gibson, J.L. Greene, J.A. Ledbetter, C. Singh, and M.A. Tepper. 1992. Immunosuppression in vivo by a soluble form of the CTLA4 T cell activation molecule. Science 257:792*.*
13. Linsley, P.S., W. Brady, M. Urnes, L.S. Grosmaire, N.K. Damle, and J.A. Ledbetter. 1991. CTLA4 is a second receptor for the B cell activation antigen B7. J.Exp.Med 174:561.
14. Bolling, S.F., H. Lin, R.Q. Wei, P. Linsley, and L.A. Turka. 1994. The effect of combination cyclosporine and CTLA4Ig therapy on cardiac allograft survival. J. Surg. Res. 57:60.
15. Judge, T.A., A. Tang, L.M. Spain, G.J. Deans, M.H. Sayegh, and L.A. Turka. 1996. The in vivo mechanism of action of CTLA4Ig. J Immunol. 156:2294.
16. Judge, T.A., Z. Wu, X.G. Zheng, A.H. Sharpe, M.H. Sayegh, and L.A. Turka. 1999. The role of CD80, CD86, and CTLA4 in alloimmune responses and the induction of long-term allograft survival. J.Immunol. 162:1947.
17. Grabstein, K.H., C.R. Maliszewski, K. Shanebeck, T.A. Sato, M.K. Spriggs, W.C. Fanslow, and R.J. Armitage. 1993. The regulation of T cell-dependent antibody formation in vitro by CD40 ligand and IL-2. J.Immunol. 150:3141.
18. Ciubotariu, R., A.I. Colovai, G. Pennesi, Z. Liu, D. Smith, P. Berlocco, R. Cortesini, and F.N. Suciu. 1998. Specific suppression of human CD4+ Th cell responses to pig MHC antigens by CD8+CD28- regulatory T cells. J.Immunol 161:5193.
19. Griggs, N.D., S.S. Agersborg, R.J. Noelle, J.A. Ledbetter, P.S. Linsley, and K.S. Tung. 1996. The relative contribution of the CD28 and gp39 costimulatory pathways in the clonal expansion and pathogenic acquisition of self-reactive T cells. J. Exp. Med. 183:801.
20. Tang, A., T.A. Judge, and L.A. Turka. 1997. Blockade of CD40-CD40 ligand pathway induces tolerance in murine contact hypersensitivity. Eur. J.Immunol. 27:3143.
21. Niimi, M., T.C. Pearson, C.P. Larsen, D.Z. Alexander, D. Hollenbaugh, A. Aruffo, P.S. Linsley, E. Thomas, K. Campbell, W.C. Fanslow, R.S. Geha, P.J. Morris, and K.J. Wood. 1998. The role of the CD40 pathway in alloantigen-induced hyporesponsiveness in vivo. J.Immunol. 161:5331.
22. Hathcock, K.S., G. Laszlo, H.B. Dickler, J. Bradshaw, P. Linsley, and R.J. Hodes. 1993. Identification of an alternative CTLA4 ligand costimulatory for T cell activation [see comments]. Science 262:905.
23. Damle, N.K., K. Klussman, G. Leytze, S. Myrdal, A. Aruffo, J.A. Ledbetter, and P.S. Linsley. 1994. Costimulation of T lymphocytes with integrin ligands intercellular adhesion molecule-1 or vascular cell adhesion molecule-1 induces functional expression of CTLA4, a second receptor for B7. J.Immunol. 152:2686.
24. Green, J.M. and C.B. Thompson. 1994. Modulation of T cell proliferative response by accessory cell interactions. Immunol.Res. 13:234.
25. Dubey, C. and M. Croft. 1996. Accessory molecule regulation of naive CD4 T cell activation. Immunol. Res. 15:114.
26. Kim, J.J., A. Tsai, L.K. Nottingham, L. Morrison, D.M. Cunning, J. Oh, D.J. Lee, K. Dang, T. Dentchev, A.A. Chalian, M.G. Agadjanyan, and D.B. Weiner. 1999. Intracellular adhesion molecule-1 modulates beta-chemokines and directly costimulates T cells in vivo. J.Clin.Invest. 103:869.
27. Ni, H.T., M.J. Deeths, W. Li, D.L. Mueller, and M.F. Mescher. 1999. Signaling pathways activated by leukocyte function-associated Ag-1-dependent costimulation. J.Immunol. 162:5183.
28. Wingett, D., K. Forcier, and C.P. Nielson. 1999. A role for CD99 in T cell activation. Cell Immunol. 193:17.
29. Hurtado, J.C., Y.J. Kim, and B.S. Kwon. 1997. Signals through 4-1BB are costimulatory to previously activated splenic T cells and inhibit activation-induced cell death. J.Immunol. 158:2600.
30. DeBenedette, M.A., A. Shahinian, T.W. Mak, and T.H. Watts. 1997. Costimulation of CD28- T lymphocytes by 4-1BB ligand J.Immunol. 158:551.
31. Hutloff, A., A.M. Dittrich, K.C. Beier, B. Eljaschewitsch, R. Kraft, I. Anagnostopoulos, and R.A. Kroczek. 1999. ICOS is an inducible T-cell co-stimulator structurally and functionally related to CD28. Nature 397:263.
32. Yoshinaga, S.K., J.S. Whoriskey, S.D. Khare, U. Sarmiento, J. Guo, T. Horan, G. Shih, M. Zhang, M.A. Coccia, T. Kohno, B.A. Tafuri, D. Brankow, P. Campbell, D. Chang, L. Chiu, T. Dai, G. Duncan, G.S. Elliott, A. Hui, S.M. McCabe, S. Scully, A. Shahinian, C.L. Shaklee, G. Van, T.W. Mak, and a. et. 1999. T-cell co-stimulation through B7RP-1 and ICOS. Nature 402:827.
33. Dong, H., G. Zhu, K. Tamada, and L. Chen. 1999. B7-H1, a third member of the B7 family, co-stimulates T-cell proliferation and interleukin-10 secretion [see comments]. Nat. Med. 5:1365.
34. Shimizu, Y., G.A. van-Seventer, K.J. Horgan, and S. Shaw. 1990. Roles of adhesion molecules in T-cell recognition: fundamental similarities between four integrins on resting human T cells (LFA-1, VLA-4, VLA-5, VLA-6) in expression, binding, and costimulation. Immunol.Rev. 114:109.
35. Deeths, M.J. and M.F. Mescher. 1999. ICAM-1 and B7-1 provide similar but distinct costimulation for CD8+ T cells, while CD4+ T cells are poorly costimulated by ICAM-1. Eur.J.Immunol. 29:45.
36. Martz, E. and S.H. Gromkowski. 1985. Lymphocyte function-associated antigens: regulation of lymphocyte adhesions in vitro and immunity in vivo. Adv.Exp.Med.Biol. 184:291*.*
37. Keizer, G.D., J. Borst, C.G. Figdor, H. Spits, F. Miedema, C. Terhorst, and J.E. De-Vries. 1985. Biochemical and functional characteristics of the human leukocyte membrane antigen family LFA-1, Mo-1 and p150,95. Eur. J. Immunol. 15:1142.
38. Lauzon, R.J., K.A. Siminovitch, and J.C. Roder. 1989. The role of T cell receptors in non-MHC-restricted cytotoxicity. Cell Immunol. 118:265.
39. Isobe, M., H. Yagita, K. Okumura, and A. Ihara. 1992. Specific acceptance of cardiac allograft after treatment with antibodies to ICAM-1 and LFA-1. Science 255:1125.
40. Isobe, M., J. Suzuki, S. Yamazaki, and M. Sekiguchi. 1996. Acceptance of primary skin graft after treatment with anti-intercellular adhesion molecule-1 and anti-leukocyte function-associated antigen-1 monoclonal antibodies in mice. Transplantation 62:411.
41. Isobe, M., J. Suzuki, S. Yamazaki, S. Horie, Y. Okubo, and M. Sekiguchi. 1997. Assessment of tolerance induction to cardiac allograft by anti-ICAM-1 and anti-LFA-1 monoclonal antibodies. J. Heart Lung Transplant. 16:1149.
42. Isobe, M., J. Suzuki, S. Yamazaki, Y. Yazaki, S. Horie, Y. Okubo, K. Maemura, and M. Sekiguchi. 1997. Regulation by differential development of Th1 and Th2 cells in peripheral tolerance to cardiac allograft induced by blocking ICAM-1/LFA-1 adhesion. Circulation 96:2247.
43. Miwa, S., M. Isobe, J. Suzuki, M. Makuuchi, M. Miyasaka, S. Yamazaki, and S. Kawasaki. 1997. Effect of anti-intercellular adhesion molecule-1 and anti-leukocyte function associated antigen-1 monoclonal antibodies on rat-to-mouse cardiac xenograft rejection. Surgery 121:681.
44. Suzuki, J., M. Isobe, S. Yamazaki, S. Horie, Y. Okubo, and M. Sekiguchi. 1997. Inhibition of accelerated coronary atherosclerosis with short-term blockade of intercellular adhesion molecule-1 and lymphocyte function-associated antigen-1 in a heterotopic murine model of heart transplantation. J. Heart Lung Transplant. 16:1141.
45. Lenschow, D.J., Y. Zeng, J.R. Thistlethwaite, A. Montag, W. Brady, M.G. Gibson, P.S. Linsley, and J.A. Bluestone. 1992. Long-term survival of xenogeneic pancreatic islet grafts induced by CTLA4Ig [see comments]. Science 257:789.
46. Azuma, H., A. Chandraker, K. Nadeau, W.W. Hancock, C.B. Carpenter, N.L. Tilney, and M.H. Sayegh. 1996. Blockade of T-cell costimulation prevents development of experimental chronic renal allograft rejection [see comments]. Proc. Natl. Acad. Sci. U.S.A. 93:12439.
47. Bolling, S.F., H. Lin, R.Q. Wei, and L.A. Turka. 1996. Preventing allograft rejection with CTLA4IG: effect of donor-specific transfusion route or timing. J. Heart Lung Transplant. 15:928.
48. Bolling, S.F., H. Lin, and L.A. Turka. 1996. The time course of CTLAIg effect on cardiac allograft rejection. J. Surg. Res. 63:320.
49. Russell, M.E., W.W. Hancock, E. Akalin, A.F. Wallace, J.T. Glysing, T.A. Willett, and M.H. Sayegh. 1996. Chronic cardiac rejection in the LEW to F344 rat model. Blockade of CD28-B7 costimulation by CTLA4Ig modulates T cell and macrophage activation and attenuates arteriosclerosis. J.Clin.Invest. 97:833.
50. Glysing, J.T.,. S.A. Raisanen, M.H. Sayegh, and M.E. Russell. 1997. Chronic blockade of CD28-B7-mediated T-cell costimulation by CTLA4Ig reduces intimal thickening in MHC class I and II incompatible mouse heart allografts. Transplantation 64:1641.
51. Kirk, A.D., D.M. Harlan, N.N. Armstrong, T.A. Davis, Y. Dong, G.S. Gray, X. Hong, D. Thomas, J. Fechner-JH, and S.J. Knechtle. 1997. CTLA4-Ig and anti-CD40 ligand prevent renal allograft rejection in primates. Proc.Natl.Acad.Sci. U.S.A. 94:8789.
52. Larsen, C.P., E.T. Elwood, D.Z. Alexander, S.C. Ritchie, R. Hendrix, B.C. Tucker, H.R. Cho, A. Aruffo, D. Hollenbaugh, P.S. Linsley, K.J. Winn, and T.C. Pearson. 1996. Long-term acceptance of skin and cardiac allografts after blocking CD40 and CD28 pathways. Nature 381:434.
53. Sun, H., V. Subbotin, C. Chen, A. Aitouche, L.A. Valdivia, M.H. Sayegh, P.S. Linsley, J.J. Fung, T.E. Starzl, and A.S. Rao. 1997. Prevention of chronic rejection in mouse aortic allografts by combined treatment with CTLA4Ig and anti-CD40 ligand monoclonal antibody. Transplantation 64:1838.
54. Konieczny, B.T., Z. Dai, E.T. Elwood, S. Saleem, P.S. Linsley, F.K. Baddoura, C.P. Larsen, T.C. Pearson, and F.G. Lakkis. 1998. IFN-gamma is critical for long-term allograft survival induced by blocking the CD28 and CD40 ligand T cell costimulation pathways. J.Immunol. 160:2059.
55. Trambley, J., A.W. Bingaman, A. Lin, E.T. Elwood, S.Y. Waitze, J. Ha, M.M. Durham, M. Corbascio, S.R. Cowan, T.C. Pearson, and C.P. Larsen. 1999. Asialo GM1(+) CD8(+) T cells play a critical role in costimulation blockade-resistant allograft rejection. J.Clin.Invest. 104:1715.
56. Larsen, C.P., D.Z. Alexander, D. Hollenbaugh, E.T. Elwood, S.C. Ritchie, A. Aruffo, R. Hendrix, and T.C. Pearson. 1996. CD40-gp39 interactions play a critical role during allograft rejection. Suppression of allograft rejection by blockade of the CD40-gp39 pathway. Transplantation 61:4.
57. Fulmer, R.I., A.T. Cramer, R.A. Liebert, and A.G. Liebert. 63 A.D. Transplantation of Cardiac Tissue into the Mouse Ear. The American Journal of Anatomy 113:273.
58. Blazar, B.R., P.A. Taylor, M.A. Panoskaltsis, G.S. Gray, and D.A. Vallera. 1995. Coblockade of the LFA1:ICAM and CD28/CTLA4:B7 pathways is a highly effective means of preventing acute lethal graft-versus-host disease induced by fully major histocompatibility complex-disparate donor grafts. Blood 85:2607.
59. Jaiswal, A.I., C. Dubey, S.L. Swain, and M. Croft. 1996. Regulation of CD40 ligand expression on naive CD4 T cells: a role for TCR but not co-stimulatory signals. Int. Immunol. 8:275.
60. Brunet, J.-F., F. Denizot, M.-F. Luciani, M. Roux-Dosseto, M. Suzan, M.-G. Mattei, P. Golstein. 1987. A new member of the immunoglobulin superfamily-CTLA4. Nature 328:267-270.
61. Harper, K., C. Balzano, E. Rouvier, M.-G. Mattei, M.-F. Luciani, P. Golstein. 1991. CTLA4 and CD28 activated lymphocyte molecules are closely related in both mouse and human as to sequence, message expression, gene structure and chromosomal location. J of Immunology 147:1037-1044.
62. Hardy, R.R., 1986. Handbook of Experimental Immunology, vol 1.: Immunochemistry pp. 13.1-13.13.
63. Sambrook et al., 1989. Molecular Cloning: A Laboratory Manual (Cold Spring Harbor Laboratory Press).
64. Kriegler M., 1993. Gene Transfer and Expression: A Laboratory Manual (W. H. Freeman and Co, New York, N.Y.).
65. Wu, 1993. Methods in Enzymology (Academic Press, New York).
66. Williams et al, 1991 PNAS 88:2726-2730.
67. Battleman et al, 1993 J Neurosci 13:94-951.
68. Carroll et al, 1993 J Cell Biochem 17E:241.
69. Lebkowski et al, U.S. Patent 5,354,678.
70. Davison and Elliott, 1993. Molecular Virology: A Practical Approach (IRL Press, New York).
71. U.S. Patent 5,824,655.
72. Dariavach et al., 1988. Eur. J. Immunol. 18:1901-1905.
73. Malik et al., 1989. Mol. and Cell. Biol. 9:2847.
74. Linsley et al., 1994. Immunity 1:793-801.
75. O'Shannessy et al., 1993. Anal. Biochem. 212:457-468.
76. Johnsson, B., et al., 1991. Anal. Biochem. 198: 268-277.
77. Khilko, S.N., et al., 1993. J. Biol. Chem. 268:15425-15434.
78. J. Greene et al., 1996, J. Biol. Chem. 271:26762-26771.
79. Hansen et al., 1980. Immunogenetics 10: 247-260.
80. Anderson et al U.S.Patent 6,113,898.
81. Yokochi et al., 1982.
82. Linsley et al, U.S Patent Number 6,090,914.
83. Linsley et al., 1992. J. Ex. Med 176: 1595-1604.
84. Linsley et al., (1995) J. Biol. Chem., 270:15417-15424.
85. Peach et al., (1994) J. Exp. Med., 180:2049-2058.
86. Metzler et al., (1997) Nature Struct. Biol., 4:527-531.
87. Wallace et al., 1994. CTLA4Ig treatment ameliorates the lethality of murine graft versus-host disease across major histocompatibility complex barriers. Transplantation 58(5):602-610.
88. Price, M. R., et al., 1986. Br. J. Cancer 54:393.
89. Gallegher, G., et al., 1993. Tumour Immunobiology, pages 63-79, Oxford University Press Inc., New York.
90. Souza, D., et al., 1999, Arthritis and Rheumatism 42:60.
91. WO 98/56417.
92. WO 95/34320.

## Claims

1. Combination of agents comprising
a first agent which blocks a CD28/CTLA4/B7-mediated signal by binding B7, wherein the first agent is a soluble CTLA4 molecule which is a fusion protein comprising the extracellular domain of CTLA4 fused to an immunoglobulin moiety;
a second agent which blocks a CD40/CD154-mediated signal by binding CD154, wherein the second agent is an anti-CD154 monoclonal antibody; and a third agent which blocks an interaction of LFA-1 with ICAM-1, ICAM-2, ICAM-3, α-actinin, filamin and/or cytohesin-1 by binding to LFA-1, wherein the third agent is an anti-LFA-1 monoclonal antibody;
for use in inhibiting a transplant rejection.

2. An agent which blocks a CD28/CTLA4/B7-mediated signal by binding B7,
wherein the agent is a soluble CTLA4 molecule which is a fusion protein
comprising the extracellular domain of CTLA4 fused to an immunoglobulin moiety;
for use in inhibiting a transplant rejection, which comprises co-administration of:
an agent which blocks a CD40/CD154-mediated signal by binding CD154, wherein the agent is an anti-CD154 monoclonal antibody; and
an agent which blocks an interaction of LFA-1 with ICAM-1, ICAM-2, ICAM-3, α-actinin, filamin and/or cytohesin-1 by binding to LFA-1, wherein the agent is an anti-LFA-1 monoclonal antibody.

3. An agent which blocks a CD40/CD154-mediated signal by binding CD154,
wherein the agent is an anti-CD154 monoclonal antibody;
for use in inhibiting a transplant rejection, which comprises co-administration of:
an agent which blocks a CD28/CTLA4/B7-mediated signal by binding B7,
wherein the agent is a soluble CTLA4 molecule which is a fusion protein comprising the extracellular domain of CTLA4 fused to an immunoglobulin moiety; and
an agent which blocks an interaction of LFA-1 with ICAM-1, ICAM-2, ICAM-3, α-actinin, filamin and/or cytohesin-1 by binding to LFA-1, wherein the agent is an anti-LFA-1 monoclonal antibody.

4. An agent which blocks an interaction of LFA-1 with ICAM-1, ICAM-2, ICAM-3, α-actinin, filamin and/or cytohesin-1 by binding to LFA-1, wherein the agent is an anti-LFA-1 monoclonal antibody;
for use in inhibiting a transplant rejection, which comprises co-administration of:
an agent which blocks a CD28/CTLA4/B7-mediated signal by binding B7,
wherein the agent is a soluble CTLA4 molecule which is a fusion protein comprising the extracellular domain of CTLA4 fused to an immunoglobulin moiety; and
an agent which blocks a CD40/CD154-mediated signal by binding CD154,
wherein the agent is an anti-CD154 monoclonal antibody.

5. Agent for use according to any one of claims 2 to 4, wherein the co-administration is sequential or concurrent administration of said agents in any order.

6. Combination of agents or agent for use according to any one of claims 1 to 5, wherein the transplant is an allograft transplant.

7. Combination of agents or agent for use according to any one of claims 1 to 6, wherein the extracellular domain of CTLA4 has an amino acid sequence which begins with methionine at position +1 and ends with aspartic acid at position +124 as shown in Figure 5, or which begins with alanine at position -1 and ends with aspartic acid at position +124 as shown in Figure 5.

8. Combination of agents or agent for use according to any one of claims 1 to 6, wherein the soluble CTLA4 molecule is a soluble CTLA4 mutant molecule.

9. Combination of agents or agent for use according to claim 8, wherein the soluble CTLA4 mutant molecule comprises a mutated extracellular domain of CTLA4 which binds a B7.

10. Combination of agents or agent for use according to claim 9, wherein the mutated extracellular domain of CTLA4 has an amino acid sequence which begins with methionine at position +1 and ends with aspartic acid at position +124 as shown in Figure 6, or which begins with alanine at position -1 and ends with aspartic acid at position +124 as shown in Figure 6.

11. Combination of agents or agent for use according to any one of claims 1 to 10, wherein the immunoglobulin moiety is an immunoglobulin constant region.

12. Combination of agents or agent for use according to any one claims 1 to 6,
wherein the fusion protein is CTLA4Ig comprising an amino acid sequence which begins with methionine at position +1 and ends with lysine at position +357 as shown in Figure 5, or which begins with alanine at position -1 and ends with lysine at position +357 as shown in Figure 5.

13. Combination of agents or agent for use according to any one claim 1 to 6,
wherein the fusion protein is L104EA29YIg comprising an amino acid sequence which begins with methionine at position +1 and ends with lysine at position +357 as shown in Figure 6, or which begins with alanine at position -1 and ends with lysine at position +357 as shown in Figure 6.

14. The use of any one of claims 1 to 13, wherein the anti-CD154 monoclonal antibody is MR1, ATCC HB-10916, ATCC HB-12055 or ATCC HB-12056.

15. The use of any one of claims 1 to 14, wherein the anti-LFA-1 monoclonal antibody is ATCC HB-9579 or ATCC TIB-213.

## Patentansprüche

1. Kombination von Mitteln, umfassend
ein erstes Mittel, das ein CD28/CTLA4/B7-vermitteltes Signal durch Binden von B7 blockiert, wobei das erste Mittel eins lösliches CTLA4-Molekül ist, das ein Fusionsprotein ist, umfassend die an einen Immunglobulinrest fusionierte extrazelluläre Domäne von CTLA4;
ein zweites Mittel, das ein CD40/CD154 vermitteltes Signal durch Binden von CD154 blockiert, wobei das zweite Mittel ein monoklonaler Anti-CD154-Antikörper ist; und
ein drittes Mittel, das eine Interaktion von LFA-1 mit ICAM-1, ICAM-2, ICAM-3, α-Actinin, Filamin und/oder Cytohesin-1 durch Binden an LFA-1 blockiert, wobei das dritte Mittel ein monoklonaler Anti-LFA-1-Antikörper ist;
zur Verwendung beim Hemmen einer Transplantatabstoßung.

2. Mittel, das ein CD28/CTLA4/B7-vermitteltes Signal durch Binden von B7 blockiert, wobei das Mittel ein lösliches CTLA4-Molekül ist, das ein Fusionsprotein ist, umfassend die an einen Immunglobulinrest fusionierte extrazelluläre Domäne von CTLA4;
zur Verwendung beim Hemmen einer Transplantatabstoßung, die gemeinsame Verabreichung von Folgendem umfasst:
ein Mittel, das ein CD40/CD154-vermitteltes Signal durch Binden von CD154 blockiert, wobei das Mittel ein monoklonaler Anti-CD154-Antikörper ist; und
ein Mittel, das eine Interaktion von LFA-1 mit ICAM-1, ICAM-2, ICAM-3, α-Actinin, Filamin und/oder Cytohesin-1 durch Binden an LFA-1 blockiert, wobei das Mittel ein monoklonaler Anti-LFA-1-Antikörper ist.

3. Mittel, das ein CD40/CD154-vermitteltes Signal durch Binden von CD154 blockiert, wobei das Mittel ein monoklonaler Anti-CD154-Antikörper ist;
zur Verwendung beim Hemmen einer Transplantatabstoßung, die gemeinsame Verabreichung von Folgendem umfasst:
ein Mittel, das ein CD28/CTLA4/B7-vermitteltes Signal durch Binden von B7 blockiert, wobei das Mittel ein lösliches CTLA4-Molekül ist, das ein Fusionsprotein ist, umfassend die an einen Immunglobulinrest fusionierte extrazelluläre Domäne von CTLA4; und
ein Mittel, das eine Interaktion von LFA-1 mit ICAM-1, ICAM-2, ICAM-3, α-Actinin, Filamin und/oder Cytohesin-1 durch Binden an LFA-1 blockiert, wobei das Mittel ein monoklonaler Anti-LFA-1-Antikörper ist.

4. Mittel, das eine Interaktion von LFA-1 mit ICAM-1, ICAM-2, ICAM-3, α-Actinin, Filamin und/oder Cytohesin-1 durch Binden an LFA-1 blockiert, wobei das Mittel ein monoklonaler Anti-LFA-1-Antikörper ist;
zur Verwendung beim Hemmen einer Transplantatabstoßung, die gemeinsame Verabreichung von Folgendem umfasst:
ein Mittel, das ein CD28/CTLA4/B7-vermitteltes Signal durch Binden von B7 blockiert, wobei das Mittel ein lösliches CTLA4-Molekül ist, das ein Fusionsprotein ist, umfassend die an einen Immunglobulinrest fusionierte extrazelluläre Domäne von CTLA4; und
ein Mittel, das ein CD40/CD154-vermitteltes Signal durch Binden von CD154 blockiert, wobei das Mittel ein monoklonaler Anti-CD154-Antikörper ist.

5. Mittel zur Verwendung nach einem der Ansprüche 2 bis 4, wobei die gemeinsame Verabreichung aufeinanderfolgende oder gleichzeitige Verabreichung der Mittel in einer beliebigen Reihenfolge ist.

6. Kombination von Mitteln oder Mittel zur Verwendung nach einem der Ansprüche 1 bis 5, wobei das Transplantat ein allogenes Transplantat ist.

7. Kombination von Mitteln oder Mittel zur Verwendung nach einem der Ansprüche 1 bis 6, wobei die extrazelluläre Domäne von CTLA4 eine Aminosäuresequenz aufweist, die mit Methionin an Position +1 beginnt und mit Asparaginsäure an Position +124 endet, wie in Figur 5 dargestellt, oder mit Alanin an Position -1 beginnt und mit Asparaginsäure an Position +124 endet, wie in Figur 5 dargestellt.

8. Kombination von Mitteln oder Mittel zur Verwendung nach einem der Ansprüche 1 bis 6, wobei das lösliche CTLA4-Molekül ein lösliches CTLA4-Mutantenmolekül ist.

9. Kombination von Mitteln oder Mittel zur Verwendung nach Anspruch 8, wobei das lösliche CTLA4-Mutantenmolekül eine mutierte extrazelluläre Domäne von CTLA4, die ein B7 bindet, umfasst.

10. Kombination von Mitteln oder Mittel zur Verwendung nach Anspruch 9, wobei die mutierte extrazelluläre Domäne von CTLA4 eine Aminosäuresequenz aufweist, die mit Methionin an Position +1 beginnt und mit Asparaginsäure an Position +124 endet, wie in Figur 6 dargestellt, oder mit Alanin an Position -1 beginnt und mit Asparaginsäure an Position +124 endet, wie in Figur 6 dargestellt.

11. Kombination von Mitteln oder Mittel zur Verwendung nach einem der Ansprüche 1 bis 10, wobei der Immunglobulinrest eine konstante Immunglobulinregion ist.

12. Kombination von Mitteln oder Mittel zur Verwendung nach einem der Ansprüche 1 bis 6, wobei das Fusionsprotein CTLA4Ig ist, umfassend eine Aminosäuresequenz, die mit Methionin an Position +1 beginnt und mit Lysin an Position +357 endet, wie in Figur 5 dargestellt, oder mit Alanin an Position -1 beginnt und mit Lysin an Position +357 endet, wie in Figur 5 dargestellt.

13. Kombination von Mitteln oder Mittel zur Verwendung nach einem der Ansprüche 1 bis 6, wobei das Fusionsprotein L104EA29YIg ist, umfassend eine Aminosäuresequenz, die mit Methionin an Position +1 beginnt und mit Lysin an Position +357 endet, wie in Figur 6 dargestellt, oder mit Alanin an Position -1 beginnt und mit Lysin an Position +357 endet, wie in Figur 6 dargestellt.

14. Verwendung nach einem der Ansprüche 1 bis 13, wobei der monoklonale Anti-CD154-Antikörper MR1, ATCC HB-10916, ATCC HB-12055 oder ATCC HB-12056 ist.

15. Verwendung nach einem der Ansprüche 1 bis 14, wobei der monoklonale Anti-LFA-1-Antikörper ATCC HB-9579 oder ATCC TIB-213 ist.

## Revendications

1. Combinaison d'agents comprenant
un premier agent qui bloque un signal médié par CD28/CTLA4/B7 en se liant à B7, ledit premier agent étant une molécule CTLA4 soluble qui est une protéine de fusion comprenant le domaine extracellulaire de CTLA4 fusionné à un groupement d'immunoglobuline ;
un deuxième agent qui bloque un signal médié par CD40/CD 154 en se liant à CD154, ledit deuxième agent étant un anticorps monoclonal anti-CD154 ; et
un troisième agent qui bloque une interaction de LFA-1 avec l'ICAM-1, l'ICAM-2, l'ICAM-3, l'α-actinine, la filamine et/ou la cytohésine-1 en se liant à LFA-1, ledit troisième agent étant un anticorps monoclonal anti-LFA-1 ;
destinée à être utilisée dans l'inhibition d'un rejet de greffe.

2. Agent qui bloque un signal médié par CD28/CTLA4/B7 en se liant à B7, ledit agent étant une molécule CTLA4 soluble qui est une protéine de fusion comprenant le domaine extracellulaire de CTLA4 fusionné à un groupement d' immunoglobuline ;
destiné à être utilisé dans l'inhibition d'un rejet de greffe, qui comprend la co-administration de :
un agent qui bloque un signal médié par CD40/CD 154 en se liant à CD 154, ledit agent étant un anticorps monoclonal anti-CD154 ; et
un agent qui bloque une interaction de LFA-1 avec l'ICAM-1, l'ICAM-2, l'ICAM-3, l'α-actinine, la filamine et/ou la cytohésine-1 en se liant à LFA-1, ledit agent étant un anticorps monoclonal anti-LFA-1.

3. Agent qui bloque un signal médié par CD40/CD154 en se liant à CD154, ledit agent étant un anticorps monoclonal anti-CD154 ;
destiné à être utilisé dans l'inhibition d'un rejet de greffe, qui comprend la co-administration de :
un agent qui bloque un signal médié par CD28/CTLA4/B7 en se liant à B7, ledit agent étant une molécule CTLA4 soluble qui est une protéine de fusion comprenant le domaine extracellulaire de CTLA4 fusionné à un groupement d'immunoglobuline ; et
un agent qui bloque une interaction de LFA-1 avec l'ICAM-1, l'ICAM-2, l'ICAM-3, l'α-actinine, la filamine et/ou la cytohésine-1 en se liant à LFA-1, ledit agent étant un anticorps monoclonal anti-LFA-1.

4. Agent qui bloque une interaction de LFA-1 avec l'ICAM-1, l'ICAM-2, l'ICAM-3, l'α-actinine, la filamine et/ou la cytohésine-1 en se liant à LFA-1, ledit agent étant un anticorps monoclonal anti-LFA-1 ;
destiné à être utilisé dans l'inhibition d'un rejet de greffe, qui comprend la co-administration de :
un agent qui bloque un signal médié par CD28/CTLA4/B7 en se liant à B7, ledit agent étant une molécule CTLA4 soluble qui est une protéine de fusion comprenant le domaine extracellulaire de CTLA4 fusionné à un groupement d'immunoglobuline ; et
un agent qui bloque un signal médié par CD40/CD154 en se liant à CD 154, ledit agent étant un anticorps monoclonal anti-CD154.

5. Agent à utiliser selon l'une quelconque des revendications 2 à 4, où la co-administration est une administration séquentielle ou concomitante desdits agents dans un ordre quelconque.

6. Combinaison d'agents ou agent à utiliser selon l'une quelconque des revendications 1 à 5, où la greffe est une greffe allogénique.

7. Combinaison d'agents ou agent à utiliser selon l'une quelconque des revendications 1 à 6, où le domaine extracellulaire de CTLA4 a une séquence d'acides aminés qui commence par une méthionine en position +1 et finit par un acide aspartique en position +124 comme le montre la figure 5, ou qui commence par une alanine en position -1 et finit par un acide aspartique en position +124 comme le montre la figure 5.

8. Combinaison d'agents ou agent à utiliser selon l'une quelconque des revendications 1 à 6, où la molécule CTLA4 soluble est une molécule CTLA4 mutante soluble.

9. Combinaison d'agents ou agent à utiliser selon la revendication 8, où la molécule CTLA4 mutante soluble comprend un domaine extracellulaire muté de CTLA4 qui se lie à un B7.

10. Combinaison d'agents ou agent à utiliser selon la revendication 9, où le domaine extracellulaire muté de CTLA4 a une séquence d'acides aminés qui commence par une méthionine en position +1 et finit par un acide aspartique en position +124 comme le montre la figure 6, ou qui commence par une alanine en position -1 et finit par un acide aspartique en position +124 comme le montre la figure 6.

11. Combinaison d'agents ou agent à utiliser selon l'une quelconque des revendications 1 à 10, où le groupement d'immunoglobuline est une région constante d'immunoglobuline.

12. Combinaison d'agents ou agent à utiliser selon l'une quelconque des revendications 1 à 6, où la protéine de fusion est CTLA4Ig comprenant une séquence d'acides aminés qui commence par une méthionine en position +1 et finit par une lysine en position +357 comme le montre la figure 5, ou qui commence par une alanine en position -1 et finit par une lysine en position +357 comme le montre la figure 5.

13. Combinaison d'agents ou agent à utiliser selon l'une quelconque des revendications 1 à 6, où la protéine de fusion est L104EA29Ylg comprenant une séquence d'acides aminés qui commence par une méthionine en position +1 et finit par une lysine en position +357 comme le montre la figure 6, ou qui commence par une alanine en position -1 et finit par une lysine en position +357 comme le montre la figure 6.

14. Utilisation selon l'une quelconque des revendications 1 à 13, dans laquelle l'anticorps monoclonal anti-CD154 est MR1, ATCC HB-10916, ATCC HB-12055 ou ATCC HB-12056.

15. Utilisation selon l'une quelconque des revendications 1 à 14, dans laquelle l'anticorps monoclonal anti-LFA-1 est ATCC HB-9579 ou ATCC TIB-213.
